(19) Europäisches Patentamt / European Patent Office / Office européen des brevets

(11) **EP 4 710 954 A1**

(12) **EUROPÄISCHE PATENTANMELDUNG**

(43) Veröffentlichungstag:
**18.03.2026 Patentblatt 2026/12**

(21) Anmeldenummer: 24315420.0

(22) Anmeldetag: **16.09.2024**

(51) Internationale Patentklassifikation (IPC):
*A61L 15/22* (2006.01)     *A61L 15/46* (2006.01)

(52) Gemeinsame Patentklassifikation (CPC):
(C-Sets verfügbar)
**A61L 15/46; A61L 15/225**          (Forts.)

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH MA MD TN**

(71) Anmelder: **Paul Hartmann AG**
**89522 Heidenheim (DE)**

(72) Erfinder:
• **Kettel, Markus**
**89522 Heidenheim (DE)**
• **Karl, Michael Maximilian**
**89522 Heidenheim (DE)**
• **Vrana, Nihal Engin**
**67000 Strasbourg (FR)**
• **Skander, Hathroubi**
**67000 Strasbourg (FR)**

(54) **ABSORBIERENDE WUNDABDECKUNG MIT INFEKTIONSHEMMENDEN EIGENSCHAFTEN**

(57) Die vorliegende Erfindung betrifft eine Wundabdeckung umfassend mindestens ein Kunststoffnetz und ein absorbierendes Vliesmaterial, wobei zumindest das Netz eine antimikrobielle Beschichtung aufweist. Die Beschichtung umfasst eine Hyaluronsäure und ein Polypeptid ausgewählt aus Polyarginin, Polylysin und Polyornithin oder einer Mischung aus mindestens zwei der zuvor genannten Polypeptide. Darüber hinaus werden Verfahren zum Auftragen der Beschichtung sowie Wundabdeckungen umfassend den antimikrobiellen Vliesstoff beschrieben. Die Beschichtung zeichnet sich durch ihre gute Verträglichkeit und Zellkompatibilität bei gleichzeitig starker antiseptischer Wirkung aus.

**Pseudomonas aeruginosa**

Figur 2a

EP 4 710 954 A1

(52) Gemeinsame Patentklassifikation (CPC): (Forts.)

C-Sets
**A61L 15/225, C08L 5/08;**
**A61L 15/225, C08L 77/04**

**Beschreibung**

**Technisches Gebiet der Erfindung**

Die vorliegende Erfindung betrifft die Wundbehandlung, insbesondere die Behandlung akuter sowie infizierter Wunden. Hierzu gehören beispielsweise Schürfwunden und auch klaffende Wunden wie Schnittverletzungen und postoperative Wunden.

**Hintergrund der Erfindung**

Akute Wunden benötigen eine schnelle und unkomplizierte Versorgung, um die Wundheilung zu unterstützen, die Vermehrung möglicher Erreger zu hemmen und die Wunde von äußeren Stressfaktoren abzuschirmen. Risikofaktoren können zum einen im physiologischen Gesamtzustand der verletzten Person liegen (hohes Alter, Diabetes, AIDS, Immunsuppression, Durchblutungsstörungen etc.), in der Umgebung (Staub, Schmutz, UV-Strahlung etc.) oder aber in der Art der Wunde, wobei gerade Schnittverletzungen bis in tiefe Gewebeschichten reichen können, was von außen nicht immer sichtbar ist (z.B. postoperative Wunden).

Werden akute Wunden nicht oder nicht korrekt versorgt, drohen Komplikationen. Bereits geschlossene Wunden können beispielsweise erneut aufreißen. Das größte Schadpotential geht jedoch von einer möglichen Infektion aus. Pathogene Keime können beim Entstehen der Wunde in die exponierten Gewebebereiche eindringen (z.B. Schürfwunden, Bisswunden) oder im Nachgang in die Verletzung gelangen. Besonders problematisch sind hierbei sogenannte nosokomiale Infektionen ("Krankenhausinfektionen"). In diesem Zusammenhang gehören die beiden Bakterienarten *Staphylococcus aureus* und *Pseudomonas aeruginosa* zu den gefährlichsten Erregern. *P. aeruginosa* besitzt die Fähigkeit zur Hämolyse. Das Bakterium produziert mehrere unterschiedliche Toxine und ist Teil der "WHO priority list for antibiotic resistant bacteria". Die Behandlung mit konventionellen Antibiotika gestaltet sich in der Regel schwierig bis unwirksam. Deswegen wird in vielen Fällen auf Kombinationstherapien und / oder Reserveantibiotika zurückgegriffen. Das eben Gesagte gilt auch für S. *aureus,* wobei dieses Bakterium zusätzlich über effektive Mechanismen verfügt, sich vor Abwehrmaßnahmen des Immunsystems zu schützen. Weiterhin ist dieser Erreger in der Lage in menschliche Zellen einzudringen und Gewebe zu zersetzen. Seine Toxine können ein toxisches Schocksyndrom auslösen. Ferner können Infektionen mit S. *aureus* eine Blutvergiftung nach sich ziehen.

Aus dem Stand der Technik sind Wundauflagen mit antibiotischer Wirksamkeit bekannt. Diese beruhen in der Regel auf der Verwendung körperfremder antimikrobieller Wirkstoffe. Derartige Wundauflagen zeigen zwar eine Wirkung gegen pathogene Mikroorganismen, haben jedoch die nachteilige Eigenschaft auch körpereigene Zellen zu beeinträchtigen. Mittels *in vitro* Assays konnte eine erhöhte Zytotoxizität für tierische Zellen nachgewiesen werden. Die betroffenen Zellen werden gestresst und ihre Vitalwerte sinken ab. Bei Testkonzentrationen, die den Realbedingungen nahekommen, stirbt für gewöhnlich ein Teil der im Assay befindlichen Zellen ab.

Die EP 1 755 569 B9 beschreibt eine Wundauflage mit Salbe, die zusätzlich ein antibakterielles Metall wie beispielsweise Silber enthält. Allerdings geht die Wirkung von Silber mit deutlich messbarer Zytotoxizität einher.

Die EP 3 452 118 B1 beschreibt antimikrobielle Beschichtungen, die Hyaluronsäure und ein Polypeptid enthalten, wobei die Säure und das Peptid nicht durchmischt vorliegen. Spezifische Ausgestaltungen von Wundauflagen werden nicht genannt.

Die EP 2 371 335 B1 beschreibt eine antibakterielle Wundauflage mit dem Wirkstoff Polyhexamethylenbiguanid (PHMB). Jüngste Untersuchungen haben jedoch gezeigt, dass PHMB problematischer ist, als ursprünglich angenommen wurde. So wurden toxische Effekt im *in vitro* Assay bei Humanzellen festgestellt (Medical mycology, 2017, 55. Jg., Nr. 3, S. 334-343) und auch *in vivo* Versuche an Ratten zeigten ein ernstzunehmendes Schädigungspotential (Interdisciplinary Toxicology, 2015, 8. Jg., Nr. 4, S. 193-202).

Darüber hinaus eigenen sich die aus dem Stand der Technik bekannten antibakteriellen Wundauflagen nur bedingt für klaffende Wunden, da die verwendeten Materialien wenig elastisch sind und nicht im vorgedehnten Zustand über die klaffenden Wundränder gelegt werden können. Folglich verfügen diese Produkte nicht über die gewünschte Rückstellkraft.

Ein weiteres ungelöstes Problem liegt darin, dass bekannte antibakterielle Wundauflagen keine oder keine ausreichenden atraumatischen Eigenschaften aufweisen, so dass deren Wundkontaktfläche während des Heilungsprozesses sich mit der Wunde verbindet und beim Abnehmen oder Wechseln des Verbands ein Aufreißen droht. Dieser Effekt kann beispielsweise dadurch zustande kommen, dass geronnenes Blut, eingetrocknetes Wundexsudat oder gebildeter Schorf das Ablösen der Wundauflage erschweren.

Folglich besteht ein Bedarf nach einem Mittel zur Wundabdeckung, das ausreichende antimikrobielle Wirksamkeit zeigt, nicht cytotoxisch für menschliche Zellen ist, Wundränder zusammenhält und zudem atraumatisch und schmerzfrei entfernt werden kann. Um dem Alltag gerecht zu werden, sollte das Mittel der Wahl zudem unmittelbar einsetzbar sein, bei längerer Lagerung über eine ausreichende Stabilität verfügen und beständig gegenüber möglichen Temperaturschwankungen sein.

**Zusammenfassung der Erfindung**

Die oben genannte Aufgabe wird gelöst durch eine Wundabdeckung umfassend

- ein Kunststoffnetz,
- ein absorbierendes Vliesmaterial,

wobei das Netz auf seiner von der Wunde abgewandten Seite von dem Vliesmaterial überlagert wird, und wobei zumindest das Netz wundseitig eine teilweise oder vollständige antimikrobielle Beschichtung aufweist, welche i) eine Hyaluronsäure sowie ii) ein Polypeptid ausgewählt aus Polyarginin, Polylysin und Polyornithin oder eine Mischung aus mindestens zwei der zuvor genannten Polypeptide umfasst.

Die erfindungsgemäße Wundabdeckung hat hervorragende atraumatische Eigenschaften, d.h. sie verbindet sich nicht mit der Wunde oder Wundbestandteilen. Weder kann Gewebe in die Wundabdeckung einwachsen, noch kann das absorbierende Vliesmaterial mit dem Wundbett verkleben. Der für gewöhnlich notwendige Einsatz von Salbe oder Silikongel zum Erreichen der atraumatischen Eigenschaften ist bei der vorliegenden Erfindung nicht notwendig, da diese Rolle von der antimikrobiellen Beschichtung und dem Kunststoffnetz erfüllt wird. Schließlich ist die Beschichtung aufgrund ihrer antimikrobiellen Inhaltsstoffe gegen humanpathogene Bakterien wirksam. Die Wundauflage kann dabei für die Behandlung verschiedener Wundarten spezifisch zusammengestellt werden, um die bestmögliche Versorgung zu gewährleisten.

Die erfindungsgemäße Wundabdeckung kann auch dann zum Einsatz kommen, wenn sich in der zu behandelnden Wunde antibiotikaresistente Bakterien befinden. Während Antibiotika üblicherweise von resistenten Bakterien beispielsweise abgebaut werden, ist ein derartiges Auftreten von Resistenzen im Hinblick auf die antimikrobielle Beschichtung der vorliegenden Erfindung nicht beobachtet worden.

Weiterhin kann die erfindungsgemäße Wundabdeckung wundheilungsfördernde Eigenschaften aufweisen. Dies ergibt sich durch die feuchtigkeitsregulierenden Eigenschaften der Hyaluronsäure, die auch *in vivo* im Bereich der extrazellulären Matrix zu finden ist.

Nachfolgend wird erläutert, wie die Wundabdeckung und die zugehörige Beschichtung strukturell und chemisch beschaffen sein können, um in der Praxis den größtmöglichen Nutzen zu erbringen.

**Detaillierte Beschreibung der Erfindung**

Der Begriff "medizinisch akzeptables Material" im Sinne der Erfindung ist eine ungiftige, fusselfreie und stabile Substanz (z.B. ein Substrat), die sich unter Normalbedingungen weder in polaren noch in unpolaren Verbindungen löst und weder von den Absonderungen tierischer noch bakterieller Zellen in nennenswertem Ausmaß abgebaut oder verflüssigt werden kann.

Der Begriff "Vliesmaterial" meint eine Schicht von miteinander zusammenhängenden Fasern, die nicht gewebt sind und in der Regel nicht nach einem sich wiederholendem Muster angeordnet sind.

Der Ausdruck "Kolonie bildende Einheit" (CFU) meint eine einzelne teilungsfähige Zelle eines Einzellers, insbesondere eines humanpathogenen Einzellers oder Bakteriums.

"Beschichtet" meint, dass eine Oberfläche eines Festkörpers (z.B. eines Netzes oder Vliesmaterials) mittels einer Substanz, die sich von der Struktur des Festkörpers unterscheidet, zumindest teilweise bedeckt bzw. überlagert wird. Insbesondere kann die Beschichtung faserlos und / oder gelartig sein. Somit kann es sich bei der Beschichtung um ein Gel, insbesondere ein hydrophiles Gel handeln.

Der Ausdruck "atraumatisch" meint, dass ein Wundversorgungsprodukt sich nicht mit der Wunde verbindet oder mit der Wunde verklebt, also beispielsweise nicht in der Wunde eintrocknet oder mit dieser verwächst und dass eine schmerzfreie Entfernung des Produktes ohne Störung des Heilungsprozesses möglich ist.

Der Ausdruck "antimikrobielle Inhaltsstoffe" meint Hyaluronsäure und ein oder mehrere Polypeptide, bei denen es sich um Polyarginin und / oder Polylysin und / oder Polyornithin handelt.

Der Ausdruck "antimikrobiell" meint im weitesten Sinne, dass ein Wirkstoff, Wirkstoffgemisch oder ein damit behandelter Artikel (z.B. eine Wundabdeckung) in der Lage ist, die Vermehrung von Mikroorganismen zu hemmen oder aufzuhalten oder die Anzahl vitaler Mikroorganismen zu reduzieren. Im engeren Sinne ist gemeint, dass ein solcher Artikel in der Lage ist, in einem Test nach ISO 20743:2021 die Anzahl der CFU des *Pseudomonas aeruginosa* Stammes mit der Hinterlegungsnummer ATTC 27853 und / oder des *Staphylococcus aureus* Stammes mit der Hinterlegungsnummer ATTC 25923 um mindestens drei Logarithmusstufen, bevorzugt um mindestens vier Logarithmusstufen, besonders bevorzugt um mindestens fünf Logarithmusstufen und ganz besonders bevorzugt um mindestens sechs Logarithmusstufen im Vergleich zu einem baugleichen Artikel ohne Wirkstoff zu reduzieren. Darüber hinaus schließt der Ausdruck "antimikrobiell" den Ausdruck "antibakteriell" mit ein.

Die Ausdrücke "Aminosäure" und "Aminosäuren" beziehen sich - sofern nicht anders angegeben - auf die Verbindungen Arginin, Lysin und / oder Ornithin, welche allesamt zu den positiv geladenen Aminosäuren zählen. Dies schließt insbesondere die L-Enantiomere der genannten Aminosäuren ein.

Die Ausdrücke "Polypeptid" und "antimikrobielles Polypeptid" meinen vorliegend Polyarginin, Polylysin und / oder Polyornithin und beziehen sich auf Peptidverbindungen, die mindestens 11, bevorzugt mindestens 20, besonders bevorzugt mindestens 30 Untereinheiten in Form von verbundenen Aminosäuren umfassen. Im Rahmen der Erfindung können die Aminosäuren innerhalb eines solchen Polypeptids allesamt identisch sein (selbe Aminosäure). Alternativ können Mischungen aus zwei oder drei der zuvor genannten Aminosäuren innerhalb eines Polypeptids vorliegen. Alle derartigen Polypeptide haben eine positive Nettoladung. Dem Fachmann ist bekannt, dass sich für die Aminosäuren am C-terminalen sowie N-terminalen Ende naturgemäß strukturelle Unterschiede ergeben, da diese Positionen die jeweiligen Kettenenden darstellen.

Der Ausdruck "Polylysin" umfasst die stereochemische Variante $\alpha$-Poly-L-Lysin und / oder $\varepsilon$-Poly-L-Lysin.

Die Ausdrücke "abwechseln" bzw. "abwechselnd" sowie "alternierend" meinen, dass auf eine erste Lage enthaltend Hyaluronsäure eine zweite Lage enthaltend das Polypeptid oder die Polypeptide folgt. Eine optionale dritte Lage würde folglich wieder Hyaluronsäure enthalten. Auf diese Wiese wechseln sich die Lagen in ihrer Nettoladung ab und bilden aufgrund ihrer Anziehungskräfte eine stabile Beschichtung aus. Alternativ kann in diesem Sinne natürlich auch auf eine erste Lage enthaltend das Polypeptid oder die Polypeptide eine zweite Lage enthaltend die Hyaluronsäure usw. folgen.

Der Ausdruck "proximal" bedeutet, dass ein Element im Einsatz zur Wunde oder Haut hin ausgerichtet bzw. wundzugewandt ist.

Der Ausdruck "distal" bedeutet, dass ein Element im Einsatz von der Wunde oder Haut weg zeigt bzw. wundabgewandt ist.

Der Ausdruck "Wundabdeckung" meint im Sinne der Erfindung ein Produkt zur Versorgung von Wunden durch deren Abdeckung, das dazu geeignet ist, wundseitig mit der erfindungsgemäßen antimikrobiellen Beschichtung versehen zu werden.

Die vorliegende Erfindung betrifft eine Wundabdeckung. Diese Wundabdeckung kann allein verwendet werden oder nach dem Anbringen auf eine Wunde bei Bedarf durch einen Sekundärverband überlagert werden oder mittels Sekundärverband, Klebefolie, Klebestreifen oder anderer Fixiermittel am Ort der Wunde befestigt werden. Die antimikrobielle Beschichtung der Wundabdeckung ist im Einsatz zur Wunde hin ausgerichtet ist und ist damit ein Teil der Wundkontaktschicht.

Vorteilhaft ist, dass die Wundabdeckung sowohl mittels der enthaltenen Beschichtung als auch mittels des Kunststoffnetzes atraumatisch ist und nicht mit der Wunde verklebt. Dies ermöglicht ein schmerzfreies und unkompliziertes Ablösen des Verbandsmaterials. Dabei bilden das Netz und die Beschichtung gemeinsam die atraumatische Wundkontaktschicht aus. Gleichzeitig erlaubt die Beschichtung eine leichte Initialhaftung, und zwar insbesondere an trockenem Gewebe. Dies erleichtert das Anbringen der beschichteten Wundabdeckung im Bereich der Wunde, denn in den meisten Fällen muss das Material nicht bis zur Applikation eines Fixiermittels an der Wundstelle festgehalten werden, sondern haftet zunächst von allein, so dass dem Anwender beide Hände für die Vorbereitung eines Fixiermittels (z.B. Klebestreifen oder Klebefolie) zur Verfügung stehen. Daneben ist auch die Ausstattung der Wundabdeckung mit Klebekomponenten möglich. Eine mögliche Ausgestaltung einer solchen Wundauflage ist der sogenannte Inselverband mit umlaufendem Kleberand. Dieser Kleberand kann mittels hautkompatibler Klebstoffe bereitgestellt werden, wobei Acrylkleber, Silikonkleber und synthetischer Kautschuk zu den gebräuchlichsten Mitteln gehören. Diese haben gleichzeitig den Vorteil restlos ablösbar zu sein.

Die erfindungsgemäße Wundabdeckung eignet sich für akute Wunden sowie für infizierte Wunden. Insbesondere Schürfwunden und klaffende Wunden können hervorragend vorsorgt werden. Beispiele für mögliche klaffende Wunden sind Schnittwunden und postoperative Wunden. In der Praxis treten häufig Mischformen dieser Wunden auf, z.B. infizierte klaffende Wunden oder infizierte Schürfwunden. In solchen Fällen zeigen sich die vorteilhaften Eigenschaften der erfindungsgemäßen Wundabdeckung besonders deutlich. Daneben hat die antibakterielle Beschichtung auch bei nicht infizierten Wunden den Vorteil, eine mögliche spätere Besiedelung mit Erregern zu hemmen oder zu unterbinden.

Die erfindungsgemäße Wundabdeckung umfasst die anspruchsgemäßen Bestandteile. Diese umfassen ein proximal - also zur Wunde hin - ausgerichtetes Kunststoffnetz, welches von einem absorbierenden Vliesmaterial überlagert wird, wobei zumindest das Netz und optional auch das Vliesmaterial wundseitig eine teilweise oder vollständige antimikrobielle - insbesondere antibakterielle - Beschichtung aufweisen, die antimikrobielle Inhaltsstoffe enthält. Weitere Bestandteile können bei Bedarf hinzugefügt werden, um die Wundabdeckung an den vorgesehenen Einsatzzweck anzupassen. Dies wird an anderer Stelle näher erläutert.

Im Rahmen der vorliegenden Erfindung kann vorgesehen sein, dass sich die Beschichtung ausschließlich auf der proximalen Seite der Wundabdeckung befindet und damit auf derjenigen Seite, welche in Benutzung der Wunde zugewandt ist. Diese Ausführung hat den ökonomischen Vorteil, dass Beschichtungsmaterial eingespart wird, ohne dass die Versorgung der Wunde darunter leidet. Dabei kann die Beschichtung sich zumindest auf der proximalen Seite des Netzes und optional auch auf der proximalen Seite des absorbierenden Vliesmaterials befinden.

Das Vliesmaterial ist ein flexibler und elastischer Festkörper, der sich der Form der Körper- oder Wundoberfläche anpassen kann und Fasern enthält. Es handelt sich um ein festes, unlösliches, medizinisch akzeptables Material. Das Material kann über eine Kristallgitterstruktur verfügen oder alternativ als teilkristalliner oder amorpher Festkörper vorliegen (z.B. als amorpher Thermoplast wie Polyvinylchlorid). Bei dem Material kann es sich um ein Polymer- oder um eine Polymermischung handeln, insbesondere aus einem thermoplastischen Polymer oder einer Mischung thermoplastischer Polymere.

In der Regel liegt das Vliesmaterial als mindestens eine Schicht faserhaltigen Materials vor. Bevorzugt ist das Vliesmaterial flach bzw. planar, so dass es eine im Wesentlichen einheitliche Dicke aufweist und weder die proximale noch die distale Seite nennenswerte (z.B. makroskopische) Unebenheiten oder Erhebungen aufweisen. Geringfügige produktionsbedingte Toleranzen sind hierbei zu vernachlässigen, so dass im Sinne der Erfindung eine einheitliche Dicke auch bei Abweichungen von +/- 5 % als gegeben gelten soll. Ein flach ausgebildetes Vliesmaterial kann in der Draufsicht eine rechteckige, quadratische, ovale oder runde Form haben, wobei ovale oder runde Formen sich besonders für Wunden an Gelenken eignen und rechteckige oder quadratische Formen eine effizientere Ausbeute von Bahnenmaterial (z.B. mittels Schneiden oder Stanzen) ermöglichen und auch eine bessere Nutzung von Lagerplatz erlauben.

Daneben sind auch Ausgestaltungen in Form von Tamponaden - beispielsweise in Form eines Zylinders - möglich, um tiefe Wunde (Kavitäten) auszutamponieren. Für die Tamponade von Wunden sollte bevorzugt die gesamte (nach außen zeigende) Oberfläche zumindest des Netzes mit der antimikrobiellen Beschichtung versehen sein, um die Kontaktfläche der Beschichtung zum Gewebe zu maximieren.

Im Rahmen der Erfindung umfasst zumindest das Netz und optional auch das Vliesmaterial eine antimikrobielle Beschichtung, die während der Benutzung zur Wunde ausgerichtet ist. Bevorzugt handelt es sich bei der Beschichtung um eine gleichmäßige oder im Wesentlichen gleichmäßige Verteilung einer Beschichtungsmasse, bei der jeder beschichtete Bereich des Netzes und optional auch des Vliesmaterials der Wundabdeckung mit derselben oder im Wesentlichen derselben Menge an Beschichtung ausgestattet ist.

Die erfindungsgemäße Beschichtung enthält mindestens die hierin beschriebene Hyaluronsäure und das hierin beschriebene Polypeptid bzw. die Polypeptide. Weitere Verbindungen oder strukturelle Komponenten können ebenfalls Teil der Beschichtung sein oder mit dieser kombiniert werden. Diese weiteren Verbindungen oder strukturellen Komponenten können flüssig oder fest sein. Darüber hinaus können sie positiv geladen, negativ geladen oder ladungsneutral sein.

In diesem Sinne kann die Beschichtung eine polare Flüssigkeit enthalten. Bei der polaren Flüssigkeit kann es sich um Wasser handeln. Das Wasser kann als destilliertes oder deionisiertes Wasser vorliegen. Bevorzugt liegt die polare Flüssigkeit (z.B. Wasser) als wässrige Pufferlösung vor. Beispiele für wässrige Puffer sind Citratpuffer, Ringerlösung, TRIS-Puffer, TE-Puffer, TBS-Puffer und TBS-T-Puffer. Bevorzugt handelt es sich bei dem Puffer um Tris-NaCl-Puffer. Die Konzentration des Puffers im Lösungsmittel (z. B. Wasser) kann beispielsweise 5 mmol bis 300 mmol betragen, bevorzugt beträgt die Konzentration 10 mmol bis 200 mmol. Im Falle von Tris-NaCl-Puffer kann die Konzentration von Tris beispielsweise 5 bis 300 mmol und die Konzentration von NaCl 10 mmol bis 300 mmol betragen. Alternativ kann die Konzentration des Puffers so gewählt werden, dass die gepufferte Lösung einen pH von 6,8 bis 7,8, bevorzugt 7,2 bis 7,6 hat. Diese pH-Werte beziehen sich zunächst auf die Lösung vor ihrer Vermengung mit anderen Bestandteilen der Beschichtung. Allerdings sind diese Werte auch auf die fertige Beschichtung im finalen Produkt - also der erfindungsgemäßen Wundabdeckung - anwendbar, und zwar sowohl vor als auch nach einer optionalen Trocknung.

Die Masse der Puffersubstanzen in Form einer Base oder einer Säure sowie einem geeigneten Salz dieser Base oder Säure kann ein Flächengewicht in der Beschichtung von beispielsweise 50 ng bis 1.000 ng / cm$^2$ Kunststoffnetz ausmachen, wobei die Öffnungen (Perforationen) im Kunststoffnetz dabei unberücksichtigt bleiben. Bevorzugt haben die Puffersubstanzen in der Beschichtung ein Flächengewicht von 100 ng bis 500 ng / cm$^2$ Kunststoffnetz, besonders bevorzugt ein Flächengewicht von 120 ng bis 300 ng / cm$^2$ Kunststoffnetz. Bevorzugt enthält die Puffersubstanz eine Base, besonders bevorzugt handelt es sich um Tris und ganz besonders bevorzugt wird die Base Tris kombiniert mit dem Salz NaCl.

Die Beschichtung kann einen Konservierungsstoff oder einen Stabilisator in einer Menge von 0,1 bis 2 Gew.-% enthalten. Beispiele für geeignete Konservierungsstoffe sind beispielsweise Benzoesäure, Sorbinsäure oder Parabene. Beispiele für geeignete Stabilisatoren sind Ascorbyl Palmitate und Tocopherol.

Da die Wundabdeckung mitsamt Beschichtung in der Regel vor der Verwendung sterilisiert wird, kann in den meisten Fällen auf die Verwendung von Konservierungsstoffen verzichtet werden, um den Produktionsaufwand gering zu halten. Somit kann vorzugsweise vorgesehen sein, dass die Beschichtung generell keine Konservierungsstoffe und / oder Stabilisatoren enthält, um die Wahrscheinlichkeit für das Auftreten von Allergien und Unverträglichkeitsreaktionen zu reduzieren. In diesem Sinne kann die erfindungsgemäße Beschichtung und die damit ausgestattete Wundabdeckung antiallergen sein.

Der Gehalt an polaren Flüssigkeiten, insbesondere an Wasser, in der Beschichtung kann 0,1 bis 50 Gew.-%

betragen. In manchen Fällen (z.B., wenn die Beschichtung als Gel vorliegen soll) können auch mehr als 50 Gew.-% an polaren Flüssigkeiten gewünscht und sinnvoll sein. Bevorzugt enthält die Beschichtung 1 bis 45 Gew.-% polare Flüssigkeiten, besser 3 bis 40 Gew.-% polare Flüssigkeiten und am besten enthält die Beschichtung 5 bis 35 Gew.-% polare Flüssigkeiten. Diese Konzentrationen können sich auf den finalen Gehalt im fertigen Produkt nach aktiver oder passiver Trocknung beziehen. Es können zwei oder mehr verschiedene polare Flüssigkeiten in der Beschichtung vorhanden sein. Beispiele für mögliche Kombinationen aus polaren Flüssigkeiten sind Wasser und Ethanol oder Wasser und Glycerin. Darüber hinaus kann es sich bei der polaren Flüssigkeit um eine flüssige Pufferlösung wie beispielsweise Tris-NaCl-Puffer handeln. Die polare Flüssigkeit bzw. die Pufferlösung können hierbei einen pH von 6 bis 9, bevorzugt 7 bis 8 und besonders bevorzugt 7,2 bis 7,6 haben.

Durch die Interaktion der negativ geladenen Hyaluronsäure und der positiv geladenen Peptide bietet die erfindungsgemäße Beschichtung eine hervorragende Langzeithaltbarkeit. Diese Langzeitstabilität ist bei medizinischen Artikeln eine besonders gewünschte Eigenschaft, da derartige Produkte von medizinischen Einrichtungen im Rahmen rabattierter Großbestellungen erworben werden und bis zu ihrem Einsatz (dessen Zeitpunkt im Regelfall nicht absehbar ist) gelagert werden müssen. Während der Lagerung können die Produkte durchaus saisonal bedingten Temperaturschwankungen unterliegen. Die erfindungsgemäße Beschichtung gewährleistet Beständigkeit sowohl bei langer Lagerungsdauer als auch gegenüber Temperaturschwankungen. Letzteres ist auch einem möglichen Sterilisationsprozess zuträglich.

Die Beschichtung und somit die beschichtete Wundabdeckung haben antimikrobielle, insbesondere antibakterielle Eigenschaften. Die antimikrobielle Wirkung tritt bereits beim Initialkontakt mit den Erregern ein und kann sich im Laufe der Zeit verstärken, wobei eine Kontaktzeit (z.B. Anwendungsdauer auf einer Wunde) von 0 bis 24 Stunden ein bevorzugter Wirkungszeitraum ist.

Bei dem im Rahmen der Erfindung eingesetzten Polypeptid oder Polypeptiden kann es sich um Polyarginin, Polylysin und / oder Polyornithin handeln. Somit kann jedes Polypeptidmolekül nur Aminosäuren eines einzigen Typs enthalten. Bei Polylysin kann es sich um $\alpha$-Poly-L-Lysin und / oder $\varepsilon$-Poly-L-Lysin handeln. Das Polylysin kann beispielsweise eine Molekülmasse von 3,5 bis 4 kDa haben. Weiterhin kann das Polylysin pro Molekül 11 bis 50 Untereinheiten, bevorzugt 15 bis 35 Untereinheiten und besonders bevorzugt 20 bis 30 Untereinheiten Lysin umfassen. Ebenso kann das Polyarginin pro Molekül 11 bis 50 Untereinheiten, bevorzugt 15 bis 35 Untereinheiten und besonders bevorzugt 20 bis 30 Untereinheiten Arginin umfassen.

Alle diese genannten Polypeptide tragen aufgrund ihrer chemischen Eigenschaften eine positive Nettoladung und wirken antimikrobiell. Polyarginin hat darüber hinaus zusätzlich den Vorteil die Wundheilung zu fördern, indem der Anteil sogenannter M2 Makrophagen innerhalb der Makrophagen-Population erhöht wird. Während Makrophagen vom Typ M1 Entzündungsreaktionen initiieren und die Produktion von cytotoxischen Radikalen einleiten, wirken M2 Makrophagen entzündungshemmend, proliferativ und fördern den Gewebeverschluss.

Die Anzahl der Moleküle für die unterschiedlichen Arten von Polypeptiden kann variabel sein. Auch können in der Beschichtung Polypeptide mit unterschiedlichen Kettenlängen vorhanden sein.

Weiterhin können Polypeptide Mischungen der drei zuvor genannten Aminosäuren in einem Molekül enthalten. Dabei können die Aminosäuren Polyarginin und Polylysin oder aber Polyarginin und Polyornithin in einem Polypeptid enthalten sein oder das Polypeptid enthält alle drei Aminosäuren.

Bevorzugt enthält die erfindungsgemäße Beschichtung Polyarginin. Der Einsatz derartiger erfindungsgemäßer polyargininhaltiger Beschichtungen ist selbst bei nicht infizierten Wunden vorteilhaft, da die Heilung beschleunigt wird.

Darüber hinaus bietet die Kombination aus Polyarginin und Polylysin in der Beschichtung eine besonders ausgeprägte antimikrobielle Wirkung, die vermutlich auf einen synergistischen Effekt zurückzuführen ist und die antimikrobielle Wirkung der jeweiligen Einzelsubstanz gegenüber diversen Erregern zu übertreffen vermag.

Bevorzugt kann die Beschichtung Polypeptide enthalten, die mindestens zehn Aminosäuren und/oder höchstens hundert Aminosäuren umfassen. Alternativ können die Polypeptide mindestens zwanzig und / oder höchsten achtzig Aminosäuren umfassen. Bevorzugt umfassen die Polypeptide mindestens fünfundzwanzig und / oder fünfzig Aminosäuren. Diese Anzahl an Aminosäuren kann für einen Teil der Polypeptide gelten oder kann alternativ auch für sämtliche Polypeptide in der Beschichtung gelten.

Alternativ kann ein Polypeptid oder Polypeptide mit im Wesentlichen einer einzigen Kettenlänge oder ausschließlich einer einzigen Kettenlänge vorhanden sein. Eine im Wesentlichen einzige Kettenlänge liegt dann vor, wenn mindestens 90 %, besser mindestens 95 %, am besten mindestens 98 % und am allerbesten mindestens 99 % aller in der Beschichtung enthaltenen Polypeptide dieselbe Kettenlänge haben. Eine Beschichtung enthaltend Polypeptide derselben oder im Wesentlichen derselben Kettenlänge bietet den Vorteil, dass die antibakterielle Wirkung und die Stabilität der Beschichtung sich sehr gut vorhersagen lassen.

Typischerweise sind diese Aminosäuren über Peptidbindungen miteinander verbunden. Die genannten Werte können sich auf einen Teil der Polypeptide in der Beschichtung (z.B. mindestens 90 Gew.-%) oder auf sämtliche Polypeptide in der Beschichtung beziehen.

Hyaluronsäure, auch bekannt als Hyaluronan, ist ein zu den Glycosaminoglycanen zählendes Heteropolysaccharid. Grundbaustein der Hyaluronsäure ist ein aus d-Glucuronsäure und N-Acetyl-d-glucosamin alternierend in (1→ 3)-(1→4)-β-glycosidischer Bindung aufgebautes Aminodisaccharid. Hyaluronsäure ist Teil der erfindungsgemäßen Beschichtung und trägt aufgrund ihrer chemischen Eigenschaften eine negative Nettoladung. Als negativ geladenes Polymer gehört Hyaluronsäure zu den Polyanionen. Hyaluronsäure ist wasserbindend und hat gewebsregenerierende und wundheilende Eigenschaften. Eine Möglichkeit Hyaluronsäure zu erhalten, ist diese zu synthetisieren, indem Proteine einer bakteriellen Fermentierung unterzogen werden. Durch eine anschließende Filtrierung wird reine Hyaluronsäure gewonnen. Die im Rahmen der vorliegenden Erfindung zu nutzende Hyaluronsäure ist hydrophil und somit löslich in Wasser und den meisten anderen polaren Substanzen.

Hyaluronsäure kann im Rahmen dieser Erfindung in Molmassen von ca. 50 bis ca. $10^4$ kg / mol verwendet werden, bevorzugt wird Hyaluronsäure mit einer molaren Masse von 140 bis 150 kg / mol verwendet. Im Rahmen dieser Erfindung ist es auch möglich, eine Mischung von Hyaluronsäuremolekülen unterschiedlicher Molmassen zu verwenden, wobei in diesem Fall die Molmasse als durchschnittliche Molmasse (Durchschnittsmolmasse) aller Hyaluronsäuremoleküle in der Mischung angegeben werden kann. Beispielsweise kann die durchschnittliche Molmasse 143 bis 146 kg / mol betragen. Alternativ haben alle oder im Wesentlichen alle Hyaluronsäuremoleküle in der Beschichtung dieselbe Molmasse. Eine im Wesentlichen selbe Molmasse liegt vor, wenn mindestens 90 %, besser mindestens 95 %, noch besser mindestens 98 % und am besten mindestens 99 % der Hyaluronsäuremoleküle in der Beschichtung dieselbe Molmasse haben. Generell gilt, dass geeignete Messmethoden zur Molmassenbestimmung wie z.B. die Massenspektrometrie aus dem Stand der Technik bekannt sind.

Die Hyaluronsäure kann quervernetzt oder unvernetzt vorliegen, wobei unvernetzte Hyaluronsäure bevorzugt ist. Gleichzeitig kann auch die antimikrobielle Beschichtung quervernetzt oder unvernetzt vorliegen, wobei unvernetzte Beschichtungen bevorzugt sind. Mögliche Verfahren zur Quervernetzung von Hyaluronsäure sind der Einsatz von BDDE, enzymatische Quervernetzung (z.B. mittels Transglutaminasen) oder physikalische Quervernetzung (z.B. mittels Gefrieren, Erhitzen, Ultraschall, Mikrowellen). Ein mögliches Verfahren zur Quervernetzung von Hyaluronsäure wird in der WO2010131175A1 beschrieben.

Weiterhin kann die Hyaluronsäure als Polymer einer einzigen Kettenlänge oder als Polymermischung mit unterschiedlicher Kettenlänge vorliegen. Zumindest ein Teil der Polymere weist eine Molekülmasse von mindestens 10 kDa und / oder höchstens 300 kDa auf. Bevorzugt hat zumindest ein Teil der Hyaluronsäurepolymere eine Molekülmasse von höchstens 250 kDa, besonders bevorzugt höchstens 200 kDa, ganz besonders bevorzugt höchstens 150 kDa und am besten höchstens 100 kDa. Dieser Teil kann z.B. 90 Gew.-% der Hyaluronsäure in der Beschichtung ausmachen oder die angegebenen Werte der Molekülmasse können sich alternativ auf die gesamte Hyaluronsäure in der Beschichtung beziehen.

Die erfindungsgemäße antimikrobielle Beschichtung kann mindestens zwei übereinanderliegende und miteinander verbundenen Lagen enthalten, wobei mindestens eine Lage vorhanden ist, welche das Polypeptid oder die Polypeptide enthält und eine positive Nettoladung hat sowie mindestens eine Lage, welche die Hyaluronsäure enthält und eine negative Nettoladung hat, und wobei sich bei den übereinanderliegenden Lagen jeweils eine das Polypeptid oder die Polypeptide enthaltende Lage mit einer die Hyaluronsäure enthaltenden Lage abwechselt, so dass eine Abfolge von alternierenden Lagen gebildet wird.

Die Anzahl der alternierenden Lagen kann dabei eine gerade oder ungerade Zahl sein. Eine gerade Anzahl an alternierenden Lagen ist dabei bevorzugt, weil dabei für jede negativ geladene Lage eine positiv geladene Lage zur Verfügung steht und sich die gegensätzlichen Ladungen anziehen, was zu einer besonders stabilen Beschichtung führt.

Vorzugsweise ist die Anzahl der alternierenden Lagen 20 bis 100, bevorzugt 30 bis 90, besonders bevorzugt 40 bis 80 und ganz besonders bevorzugt 50 bis 70. Dabei enthält jeweils die Hälfte der Lagen die Hyaluronsäure und die andere Hälfte der Lagen das Polypeptid oder die Polypeptide. Bei einer ungeraden Anzahl an alternierenden Lagen überwiegt bevorzugt die Anzahl derjenigen Lagen, die das Polypeptid enthalten.

Weiterhin kann die Beschichtung des Netzes und optional auch des Vliesmaterials einen Aufbau aus zwei oder mehr Lagen aufweisen. Der Begriff Lage bezieht sich auf eine Schicht innerhalb der Beschichtung. Eine Lage kann in einem Beschichtungsschritt aufgebracht werden. Die erste Lage ist auf das Netz und optional auch auf das Vliesmaterial aufgebracht, wobei hierbei üblicherweise die proximal gelegene Oberfläche behandelt wird. Jede weitere Lage - angefangen mit der zweiten Lage - wird auf die zuletzt aufgebrachte Lage aufgebracht. Dabei kann eine Lage entweder die Hyaluronsäure enthalten und hat somit eine negative Nettoladung oder kann das Polypeptid bzw. die Polypeptide und damit eine positive Nettoladung haben. Hierbei kann vorgesehen sein, dass eine Lage, die die Hyaluronsäure enthält, frei ist von dem Polypeptid bzw. den Polypeptiden und eine Lage, die das Polypeptid bzw. die Polypeptide enthält, frei ist von der Hyaluronsäure. Im Unterschied hierzu kann die Beschichtung auch durchmischte Lagen enthalten, auf welche an anderer Stelle näher eingegangen wird.

Darüber hinaus kann die Anzahl der übereinanderliegenden und miteinander verbundenen Lagen in der Beschichtung beispielsweise 10 bis 100 betragen.

Bevorzugt beträgt die Anzahl der übereinanderliegenden und miteinander verbundenen Lagen 15 bis 90, besonders bevorzugt 20 bis 80, ganz besonders bevorzugt 25 bis 70 und am besten 30 bis 60.

Eine Möglichkeit die Lagen der Beschichtung aufzutragen, besteht darin, diese mittels Tauchverfahren aufzubringen. Das Tauchverfahren ist darüber hinaus das geeignete Verfahren, um solche Lagen innerhalb der Beschichtung zu erzeugen, die jeweils entweder Hyaluronsäure ohne das Polypeptid bzw. die Polypeptide oder das Polypeptid bzw. die Polypeptide ohne die Hyaluronsäure enthalten. So ist es beim Tauchverfahren möglich zwei Lösungen in zwei separaten Kompartimenten bereitzustellen, wobei ein Kompartiment die Lösung mit der Hyaluronsäure und das andere Kompartiment die Lösung mit dem Polypeptid bzw. den Polypeptiden enthält. Auf das Netz und optional auch auf das Vliesmaterial können auf diese Weise durch abwechselndes Eintauchen in den beiden Kompartimenten Lagen aufgetragen werden, bis die Beschichtung vollständig ist.

Im Rahmen der Erfindung können die Hyaluronsäure und das Polypeptid bzw. die Polypeptide innerhalb der Beschichtung oder innerhalb einer Lage der Beschichtung jedoch auch durchmischt vorliegen. Durchmischt ist in diesem Sinne als Gemisch im chemischen Sinne zu verstehen. Das Gemisch ist hierbei bevorzugt eine Lösung, und zwar eine Lösung mit nur einer Phase. Bevorzugt sind die Hyaluronsäure und das Polypeptid bzw. die Polypeptide innerhalb der Beschichtung homogen verteilt.

Weiterhin bevorzugt ist das Polypeptid und / oder sind die Polypeptide in eine Hyaluronsäurematrix eingebettet. Eine solche Matrix kann als Hyaluronsäuregel vorliegen und Hyaluronsäure sowie Wasser enthalten. Die Hyaluronsäurematrix bildet sich aus, wenn die Beschichtung im trockenen Zustand vorliegt oder durch Trocknung in diesen übergeht, wobei eine Restfeuchtigkeit durch chemische Wechselwirkungen erhalten bleibt.

Eine durchmischte Beschichtung im Sinne des eben Genannten lässt sich durch Aufsprühen einer Lösung enthaltend Hyaluronsäure und Aufsprühen einer Lösung enthaltend das Polypeptid bzw. die Polypeptide auf das Kunststoffnetz und optional auch auf das Vliesmaterial erhalten. Die beiden Lösungen können nacheinander oder gleichzeitig gesprüht werden. Mögliche Verfahren sind in den Ausführungsbeispielen näher erläutert. Sobald eine Lage erhalten wurde und diese getrocknet oder angetrocknet ist, können weitere Lagen aufgesprüht oder mittels Tauchverfahren hinzugefügt werden.

Eine durchmischte Beschichtung kann eine oder mehrere Lagen enthalten, von denen mindestens eine Lage sowohl die Hyaluronsäure als auch das Polypeptid bzw. die Polypeptide enthält. In diesem Sinne ist eine solche durchmischte Beschichtung gänzlich oder möglicherweise nur teilweise von der Mischung durchdrungen.

Ferner können auch eine oder mehrere Lagen einer Beschichtung durchmischt sein, wobei eine solche durchmischte Lage sowohl die Hyaluronsäure als auch ein oder mehrere der besagten Polypeptide enthält.

Falls nur die wundzugewandte (proximale) Seite der Wundabdeckung beschichtet ist, können die distale oder proximale Seite der Wundabdeckung durch unterschiedliche Farbgebung als Ober- oder Unterseite für den Nutzer gekennzeichnet sein. Beispielsweise kann die Oberseite (distale Seite) grün sein, während die wundzugewandte Seite weiß ist. Auf diese Weise ist für den Nutzer erkennbar, welche Seite der Wundabdeckung auf die Wunde aufzulegen ist.

Gemäß einem Konzept der Erfindung haben das Polypeptid bzw. die Polypeptide der antimikrobiellen Beschichtung eine Molekülmasse von 1 bis 41 kDa. Bevorzugt haben das Polypeptid bzw. die Polypeptide eine Molekülmasse von 3 bis 35 kDa und besonders bevorzugt 5 bis 30kDa. Möglich ist auch, dass mindestens eine, mindestens zwei oder mindestens drei Lagen der Beschichtung ein Polypeptid bzw. Polypeptide mit einer solchen Molekülmasse hat.

Die Beschichtung des Netzes und optional auch des Vliesmaterials der Wundabdeckung kann eine Dicke von 10 nm bis 1000 nm haben. Bevorzugt hat die Beschichtung eine Dicke von 50 nm bis 900 nm, besonders bevorzugt eine Dicke von 100 nm bis 800 nm, besonders bevorzugt eine Dicke von 150 nm bis 700 nm und am besten 200 nm bis 600 nm. Die Dicke kann dabei gemessen werden von der Oberkante der Grundfläche des Netzes bzw. Vliesmaterials, und zwar ohne, dass das Netz bzw. das Vliesmaterial während der Messung gestaucht oder komprimiert wird. Die Werte beziehen sich auf die Dicke der Beschichtung nach aktiver oder passiver Rücktrocknung. Falls sowohl Netz als auch Vliesmaterial beschichtet sind, beziehen sich die angegebenen Werte für die Dicke der Beschichtung jeweils unabhängig voneinander auf das Netz für die Netzbeschichtung und auf das Vliesmaterial für die Vliesbeschichtung.

Es wird empfohlen die Dicke der Beschichtung durch die Anzahl der Lagen einzustellen. Die Dicke nimmt mit zunehmender Anzahl der Lagen weiter zu. Beschichtungen, deren Dicke eine größere Ausdehnung erreicht, haben besonders ausgeprägte atraumatische Eigenschaften. Beschichtungen, die eine geringe Dicke aufweisen, ermöglichen einen schnelleren Durchfluss von Flüssigkeiten aus der Wunde in die Wundabdeckung.

Das Netz und optional auch das Vliesmaterial der Wundabdeckung kann die Beschichtung mit einem Flächengewicht von beispielsweise 5 ng bis 200 ng / cm² enthalten. Bevorzugt hat die Beschichtung ein Flächengewicht von 10 ng bis 150 ng / cm², besonders bevorzugt ein Flächengewicht von 15 ng bis 130 ng / cm², ganz besonders bevorzugt ein Flächengewicht von 20 ng bis 100 ng / cm² und am besten 20 ng bis 50 ng / cm². Das Flächengewicht der Beschichtung bezieht sich dabei auf die Summe der Massen aus Polypeptid bzw. Polypeptiden und Hyaluronsäure. Bei der Bestimmung des Flächengewichts können die Perforationen im Netz unberücksichtigt bleiben. Beispielsweise kann bei einem Netz mit einer Fläche von 10 cm x 10 cm davon ausgegangen werden, dass die Fläche des Netzes 100 cm² beträgt.

Ebenso kann das Flächengewicht des Polypeptids bzw. der Polypeptide in der Beschichtung beispielsweise 1 ng bis 500 ng / cm², bevorzugt 2 ng bis 300 ng / cm² und ganz besonders bevorzugt 3 ng bis 50 ng / cm² betragen.

Ebenso kann das Flächengewicht der Hyaluronsäure in der Beschichtung beispielsweise 3 ng bis 1.000 ng / cm², bevorzugt 6 ng bis 400 ng / cm$^2$ und besonders bevorzugt 7 ng bis 50 ng / cm$^2$ betragen.

Das Verhältnis von Polypeptid bzw. Polypeptiden zu Hyaluronsäure in der Beschichtung kann beispielsweise ein Massenverhältnis von 0,5 zu 1 bis 5 zu 1 haben. Bevorzugt liegt das Verhältnis bei 1 zu 1 bis 4 zu 1, besonders bevorzugt liegt das Verhältnis bei 1,5 zu 1 bis 3,5 zu 1 und ganz besonders bevorzugt bei 2 zu 1 bis 3,5 zu 1.

Das Netz und optional auch das Vliesmaterial der Wundabdeckung können jeweils teilweise oder vollständig mit der Beschichtung beschichtet sein. Insbesondere können mindestens 80 % der Fläche der wundzugewandten (proximalen) Seite des Netzes und optional des Vliesmaterials beschichtet sein. Bevorzugt sind 90 % der Fläche der wundzugewandten Seite des Netzes und optional des Vliesmaterials beschichtet. Besonders empfohlen wird mindestens 99 % dieser Fläche zu beschichten. Netzperforationen können bei der Flächenbestimmung unberücksichtigt bleiben.

Weiterhin ist die erfindungsgemäße Beschichtung vorzugsweise trocknungsbeständig. In diesem Sinne können die Bestandteile der Beschichtung - wie Hyaluronsäure und Polypeptid oder Polypeptide - im feuchtem Zustand als Lösung auf das Netz und optional das Vliesmaterial der Wundabdeckung aufgetragen werden, um anschließend zu trocknen oder getrocknet zu werden. Das Trocknen kann passiv bei Raumtemperatur erfolgen oder aktiv unter Einsatz technischer Hilfsmittel, wobei letzteres im Allgemeinen deutlich schneller, aber auch energieaufwändiger ist. Die Beschichtung behält auch im getrockneten Zustand ihre Eigenschaften - insbesondere die atraumatische und antimikrobielle Wirkung - bei. Die Trocknungsbeständigkeit der Beschichtung hat den Vorteil, dass sich trockene Wundversorgungsprodukte einfacher im industriellen Maßstab verarbeiten und verpacken lassen. Darüber hinaus lassen sich trocknungsbeständige Wundversorgungsprodukte leichter und länger lagern, da sie nicht gegen ein ungewolltes Eintrocknen geschützt werden müssen.

Eine Restfeuchtigkeit kann auch in der getrockneten bzw. trockenen Beschichtung aufgrund der hygroskopischen Eigenschaften der Inhaltsstoffe vorhanden sein. Die Beschichtung gilt beispielsweise als trocken, wenn der Flüssigkeitsgehalt (z.B. Wassergehalt) in der Beschichtung maximal 5 Gew.-%, bevorzugt maximal 4 Gew.-%, besonders bevorzugt maximal 3 Gew.-%, ganz besonders bevorzugt maximal 1 Gew.-% und am besten maximal 0,1 Gew.-% beträgt.

Überraschenderweise hat sich gezeigt, dass die Beschichtung durchleitend für Blut und wässrige Wundflüssigkeiten wie Wundexsudat ist. Das heißt, dass Blut und Wundexsudat die Beschichtung passieren können. Auf diese Weise können diese Flüssigkeiten das Netz passieren und in das absorbierende Vliesmaterial aufgenommen werden. Sie können auch an weitere optional vorhandene Schichten, die sich distal zum Vliesmaterial befinden, weitergegeben werden. Mithilfe absorbierender Materialien kann dabei ein Sogeffekt und / oder Dochteffekt generiert werden, der Flüssigkeit von der wundzugewandten Seite zur distalen Seite der Wundabdeckung leitet. Dies erlaubt die Platzierung weiterer saugfähiger Materialien oberhalb (distal) vom Vliesmaterial, welche das hindurchtretende Exsudat speichern. Ein Beispiel hierfür ist eine Retentionsschicht. Es ist allgemein vorteilhaft, wenn eine solche Retentionsschicht hydrophiler ist als das darunter (in proximaler Richtung) befindliche Vliesmaterial. Dies kann beispielsweise dadurch erreicht werden, dass die Retentionsschicht superabsorbierende Fasern enthält.

Das Vliesmaterial kann die folgenden Materialien oder Materialgemische enthalten oder daraus bestehen: Kunststofffasern, Polyolefin basierte Fasern, Polyethylen, Polyetheretherketon (PEEK), Polyvinylchlorid (PVC), Polymethylmethacrylat (PMMA), Polykarbonat (PC), Polyester, Polyethylenterephthalat (PET), Polypropylen (PP), Polystyren (PS), Polyamid, Acrylnitril-Butadien-Styrol (ABS), Polylactide (PLA), Viskose, zellulosebasierte Fasern oder Mischungen aus zwei oder mehr der zuvor genannten. Bei Verwendung von Polyamid als Fasermaterial kann das Polyamid in Form von Nylon vorliegen. Mögliche Mischungen sind eine Mischung aus Polypropylen und Viskose, eine Mischung aus Polyethylen und Viskose, eine Mischung von Polypropylen, Polyethylen und Viskose, eine Mischung aus Polyester mit Baumwolle oder eine Mischung aus Polyester mit Viskose.

Besonders bevorzugt enthält das absorbierende Vliesmaterial der Wundabdeckung Fasern aus Polypropylen und / oder Polyethylen. Der Anteil von Polypropylen und / oder Polyethylen kann 2 Gew.-% bis 40 Gew.-% betragen. Bevorzugt beträgt der Anteil 5 Gew.-% bis 30 Gew.-%, besonders bevorzugt 7 Gew.-% bis 20 Gew.-% und ganz besonders bevorzugt 9 Gew.-% bis 16 Gew.-%. Polypropylen hat den Vorteil dem Vliesmaterial thermoplastische Eigenschaften zu verleihen. Darüber hinaus ist es äußerst reißfest. Polyethylen hat den Vorteil elastisch zu sein und ist darüber hinaus geeignet als Verstärkungsfaser zu dienen.

Bevorzugt enthält das Vliesmaterial aus mindestens zwei verschiedenen Faserarten. Das Vliesmaterial kann beispielsweise aus drei verschiedenen Faserarten bestehen. Die verschiedenen Faserarten können sich dabei in ihrer grundlegenden chemischen Struktur unterscheiden (z.B. unterschiedliche Polymere). Beispiele für mögliche Kombinationen sind Fasern natürlichen Ursprungs mit Kunstfasern, biologisch abbaubare Fasern mit Kunstfasern und Regeneratfasern mit Kunstfasern.

Bevorzugt enthält das absorbierende Vliesmaterial der Wundabdeckung Fasern aus Viskose. Der Viskoseanteil im Vliesmaterial kann 60 Gew.-% bis 98 Gew.-% betragen. Bevorzugt beträgt der Viskoseanteil 70 Gew.-% bis 95 Gew.-%, besonders bevorzugt 80 Gew.-% bis 93 Gew.-% und ganz besonders bevorzugt 84 Gew.-% bis 91 Gew.-%. Bei der Bestimmung von Gewichtsanteilen im Vliesmaterial bleiben andere Bereiche der Wundabdeckung wie Netz, Beschichtung und eine optionale Retentionsschicht generell unberücksichtigt. Die Viskose hat den Vorteil hydrophil zu sein und verleiht dem Vliesmaterial absorbierende Eigenschaften.

Weiterhin kann das absorbierende Vliesmaterial der Wundabdeckung eine Mischung aus Fasern von Viskose, Polyethylen, Polypropylen und / oder Polyethylenterephthalat enthalten.

Bevorzugt enthält das absorbierende Vliesmaterial Fasern aus Viskose, Polyethylen und entweder

a) Polypropylen oder
b) Polyethylenterephthalat,

wobei optional mindestens 80 Gew.-% des absorbierenden Vliesmaterials aus Viskose bestehen können. Bevorzugt bestehen mindestens 84 Gew.-% des absorbierenden Vliesmaterials aus Viskose, besonders bevorzugt mindestens 89 Gew.-%. Die Viskose hat als hydrophile Faser eine hervorragende Dochtwirkung und verleiht dem Vliesmaterial absorbierende Eigenschaften.

Unabhängig davon kann der Anteil von der Summe von Polyethylen und Polypropylen im Vliesmaterial mindestens 7 Gew.-%, bevorzugt mindestens 8 Gew.-%, besonders bevorzugt mindestens 9 Gew.-% und am besten mindestens 10 Gew.-% betragen. Im Falle eines Anteils von mindestens 10 Gew.-% könnte das Vliesmaterial beispielsweise 5 Gew.-% Polyethylen und 5 Gew.-% Polypropylen enthalten und der übrige Anteil könnte Viskose sein.

Die oben aufgeführten Fasern bzw. Faserarten haben als Vliesmaterial vorliegend zum einen strukturgebende Eigenschaften und darüber hinaus die vorteilhafte Eigenschaft, Flüssigkeiten wie Wundexsudat von der Wunde wegzuleiten. Dies kann durch hydrophile Eigenschaften der Fasern bewirkt werden und / oder durch physikalische Kräfte (Dochteffekt) zustande kommen. Dieser Effekt kann verstärkt werden, wenn zumindest die hydrophoben Fasern des Vliesmaterials - wie zum Beispiel Kunstfasern - einer Hydrophilierung unterzogen wurden. Die Hydrophilierung kann den Faseranteil der Fasern aus Polypropylen und / oder der Fasern aus Polyethylen betreffen.

Bei dem Vliesmaterial kann es sich um ein luftgelegtes Vliesmaterial, ein thermisch verfestigtes Vliesmaterial, ein mechanisch verfestigtes Vliesmaterial, ein Stapelfaservliesmaterial, ein Schmelzblasvliesmaterial, ein Spinnvliesmaterial, ein Vliesmaterial enthaltend Nassvliesstoff, Wirrlagevliesstoff (isotropen Vliesstoff) und / oder anisotropen Vliesstoff (orientierten Vliesstoff) handeln.

Möglich ist, dass das erfindungsgemäße Vliesmaterial ausschließlich solche Fasern enthält, die synthetischen Ursprungs sind und / oder solche Fasern, die zu den Kunstfasern gezählt werden. Viskose ist dabei eine synthetische Faser, die im Rahmen der vorliegenden Erfindung jedoch nicht zu den Kunstfasern gezählt wird.

Weiterhin ist auch möglich, dass das erfindungsgemäße Vliesmaterial ausschließlich solche Fasern enthält, die natürlichen Ursprungs sind und / oder biologisch abbaubar sind. Bei der biologischen Abbaubarkeit kann es sich um eine Abbaubarkeit im Sinne der Norm EN ISO 14851 :2019 handeln. Fasern natürlichen Ursprungs sind beispielsweise Baumwollfasern. Fasern, die im Kontext der vorliegenden Erfindung als biologisch abbaubar gelten, sind beispielsweise Baumwollfasern und Viskosefasern.

Das Vliesmaterial kann neben der erfindungsgemäßen antimikrobiellen Beschichtung weitere Verbindungen oder Strukturelemente enthalten. Hierzu gehören insbesondere Substanzen, die die Heilung fördern, die Wundränder vor Mazeration schützen und/oder die antimikrobielle Wirkung verbessern. Diese Substanzen können in die Beschichtung eingearbeitet, auf die proximale Seite der Beschichtung aufgetragen oder Teil des Vliesmaterials oder der Retentionsschicht sein. Beispiele für Substanzen mit wundheilungsfördernder Wirkung sind Allantoin oder Dexpanthenol.

Bevorzugt ist das Kunststoffnetz der Wundabdeckung faserlos ausgestaltet. Das Kunststoffnetz kann aus einem perforierten Film oder einer perforierten Folie bestehen. Durch die Abwesenheit von Fasern, hat das Netz selbst weder eine Dochtwirkung noch absorbierende Eigenschaften und deswegen kaum Interaktion mit Wundflüssigkeiten. Hierdurch erhält das Netz ausgeprägt atraumatische Eigenschaften, die durch die ebenfalls atraumatisch wirkende antimikrobielle Beschichtung weiter verstärkt werden.

Bei dem Kunststoffnetz kann es sich um ein Polymernetz handeln. Der Kunststoff kann ein reiner Kunststoff sein oder ein Gemisch aus zwei oder mehr Kunststoffen. Bevorzugt enthält der Kunststoff Polyethylen. Der Kunststoff kann beispielsweise mindestens 70-Gew.-%, mindestens 80-Gew.-%, mindestens 90 Gew.-% oder mindestens 99 Gew.-% Polyethylen enthalten. Weiterhin kann das Netz vollständig aus Polyethylen bestehen.

Es hat sich gezeigt, dass Polyethylen eine besonders geringe Interaktion mit Wunden und Wundflüssigkeiten eingeht und damit die atraumatischen Eigenschaften der Wundabdeckung steigert. Wundabdeckungen mit einem polyethylenhaltigen Netz in der Wundkontaktschicht lassen sich besonders schmerzarm nach der Anwendung entfernen. Dieser Effekt wird durch die atraumatischen Eigenschaften der antimikrobiellen Beschichtung weiter verstärkt. Daneben ist Polyethylen elastisch. Dies bietet den Vorteil, dass die Wundabdeckung im Falle von klaffenden Wunden im vorgespannten Zustand auf die Wunde aufgebracht werden kann und aufgrund der vom Polyethylen ausgehenden Rückstellkraft die Wundränder anschließend zusammengeführt werden, um die Wunde zu schließen und ein Zusammenwachsen der Wundränder zu ermöglichen.

Eine Möglichkeit ein geeignetes Kunststoffnetz bereitzustellen, besteht darin einen Kunststofffilm mittels Extrusion zu generieren und über eine mit Erhebungen versehene Perforationswalze zu führen. Durch eine optional im Anschluss folgende bidirektionale Dehnung können die Perforationen in ihrer Größe verändert werden. Nach dem Abkühlen wird ein stabiles Netz erhalten.

Die Öffnungen des Netzes können eine Fläche oder Durchschnittsfläche von 0,00001 mm$^2$ bis 4 mm$^2$ pro Öffnung haben, bevorzugt 0,007 mm$^2$ bis 1 mm$^2$. Bei kreisförmigen oder im wesentlichen kreisförmigen Öffnungen kann der durchschnittliche Durchmesser von 0,01 mm bis 1 mm, vorzugsweise von 0,1 mm bis 1 mm betragen. Öffnungen, die auf diese Weise beschaffen sind, erlauben den Durchtritt von Flüssigkeiten und vermitteln dem Netz gleichzeitig starke atraumatische Eigenschaften.

Das Netz kann auf unterschiedliche Weise mit dem Vliesmaterial verbunden werden. So können Netz und Vliesmaterial unter Einsatz eines Klebemittels aneinander befestigt werden. Eine andere Möglichkeit ist die thermische Verfestigung - beispielsweise mittels Kalanderverfestigung oder Heißluftverfestigung - oder das Schweißen. Daneben besteht die Möglichkeit das Vliesmaterial in ein allseitig umlaufendes Netz einzuschließen.

Bevorzugt ist die gesamte proximale Fläche des Vliesmaterials wundseitig vom Netz bedeckt. Ebenfalls bevorzugt ist die gesamte distale Fläche des Netzes vom Vliesmaterial überlagert. Die Netzöffnungen sind hierbei unberücksichtigt. Es hat sich gezeigt, dass es vorteilhaft ist, wenn Netz und Vliesmaterial im Wesentlichen dieselbe Ausdehnung (Fläche) haben und kongruent (deckungsgleich) zueinander ausgerichtet sind, so dass keine der beiden die jeweils andere überragt. Auf diese Weise stützen und stabilisieren sich Netz und Vliesmaterial gegenseitig und ergänzen sich darüber hinaus auch funktional.

Weiterhin kann die lineare Dichte des Vliesmaterials oder des Vliesmaterials inklusive Netz 60 bis 100 dtex, bevorzugt 70 bis 90 dtex gemessen mittels DIN EN ISO 2060 betragen. Die Zähigkeit des Vliesmaterials kann 30 bis 50 cN/tex, bevorzugt 35 bis 40 cN/tex gemessen mittels DIN EN ISO 2062 betragen.

Das Vliesmaterial oder das Vliesmaterial inklusive Netz kann eine Dehnbarkeit nach DIN EN ISO 1798:2008-04 von mindestens 25%, bevorzugt von mindestens 35% haben. Dehnbarkeit ist dahingehend zu verstehen, dass es bei einer entsprechenden Verlängerung des Materials unter Zugkraft nicht zu einem Belastungsbruch oder zu einem Reißen kommt. Bevorzugt ist die Dehnbarkeit eine reversible Dehnbarkeit, so dass das Material nach Aufhebung der Zugkraft im Wesentlichen seine ursprüngliche Länge einnimmt. Eine reversible Dehnbarkeit liegt auch dann vor, wenn das Material nach Aufhebung der Zugkraft eine Länge einnimmt, die maximal 105% der Ausgangslänge entspricht. Die Länge hat dabei dieselbe räumliche Ausrichtung wie die Zugkraft, ist also in Richtung der Zugkraft zu messen. Bevorzugt gelten die angegebenen Dehnungswerte in Faserrichtung.

Die Wundabdeckung kann distal - also oberhalb - zum Vliesmaterial eine Retentionsschicht enthalten. Falls die Wundabdeckung über ein Backing verfügt, so kann die Retentionsschicht zwischen Vliesmaterial und Backing liegen.

Vorzugsweise hat die Retentionsschicht in einem solchen Fall direkten Kontakt sowohl zum Vliesmaterial als auch zum Backing. Die Retentionsschicht hat die Aufgabe Flüssigkeit, die aus dem Vliesmaterial aufzunehmen und zu speichern. Auf diese Weise wird ein Rückfluss aufgenommener Flüssigkeit in Richtung Wunde verhindert. Zu diesem Zweck enthält die Retentionsschicht stark hydrophiles Material. Hierzu gehören a) Zellstoffflocken, welche Zellulose als Hautbestandteil enthalten können, b) superabsorbierende Partikel, c) superabsorbierende Fasern sowie d) Mischungen aus zwei oder drei der zuvor genannten Materialien. Bevorzugt enthält die Retentionsschicht superabsorbierende Fasern. Die superabsorbierenden Fasern können a) quervernetztes Acrylat-Copolymer und / oder b) eine Mischung aus Natriumacrylat, Acrylsäure und Methylacrylat umfassen. Diese chemischen Verbindungen ermöglichen eine hervorragende Quellfähigkeit, so dass die superabsorbierenden Fasern ein Vielfaches ihres eigenen Gewichts an Flüssigkeit sowohl aufnehmen als auch speichern können. Bei der Speicherung (Retention) der aufgenommenen Flüssigkeit reagiert die Flüssigkeit mit dem Superabsorber zu einer gelartigen Quellmasse, welche in der Retentionsschicht eingeschlossen ist. Durch das Aufquellen der superabsorbierenden Substanzen können Netz und Vliesmaterial in Richtung Wunde gedrückt werden, um den Kontakt zwischen antimikrobieller Beschichtung und Wundbett zu intensivieren. Dies funktioniert besonders gut, wenn die Wundabdeckung mit einem umlaufenden klebenden Backing an der Haut befestigt ist und dabei als Widerlager fungiert.

Die superabsorbierenden Fasern können 15 bis 30 Gew.-% der Retentionsschicht und / oder 2 bis 6 Gew.-% der Summe des Faseranteils von Vliesmaterial und Retentionsschicht ausmachen. Bevorzugt beträgt der Anteil superabsorbierender Fasern 18 bis 25 Gew.-% der Retentionsschicht und / oder 3 bis 5 Gew.-% der Summe des Faseranteils von Vliesmaterial und Retentionsschicht. Diese Bereiche ermöglichen eine hohe Belastbarkeit der Retentionsschicht sowohl im trockenen als auch im feuchten Zustand bei gleichzeitig hoher Absorptionskapazität.

Netz, Vliesmaterial und Retentionsschicht können zusammen als Wundkissen ausgebildet sein. In einem solchen Fall werden alle diese drei Bestandteile der Wundabdeckung durch eine durchgehende oder unterbrochene Schweißnaht zusammengehalten, welche zumindest im äußeren Randbereich (Draufsicht) ausgebildet ist.

Die Wundabdeckung kann an ihrer beschichteten Wundkontaktfläche durch eine Schutzschicht wie beispielsweise einen Release-Liner überdeckt sein. Eine solche Schutzschicht wird vom Anwender vor der Verwendung entfernt.

Ferner von der Erfindung umfasst sind Verfahren zur Herstellung der hierin beschriebenen Wundabdeckung und zum Beschichten von Wundabdeckungen mit der erfindungsgemäßen antimikrobiellen Beschichtung.

Nachfolgend wird ein Verfahren beschrieben, mit dem man eine Wundabdeckung mit durchmischter Beschichtung und / oder mit mindestens einer durchmischten Lage innerhalb der Beschichtung erzeugen kann:

a) Bereitstellen einer Wundabdeckung umfassend ein Kunststoffnetz und ein absorbierendes Vliesmaterial, wobei das Netz auf seiner von der Wunde abgewandten Seite von dem Vliesmaterial überlagert wird,

b) Besprühen des Netzes aus Kunstfasern mit i) einer Hyaluronsäure und ii) einem Polypeptid zur Ausbildung einer Beschichtung umfassend mindestens eine Beschichtungslage, wobei das Polypeptid ausgewählt ist aus Polyarginin, Polylysin und Polyornithin oder einer Mischung aus mindestens zwei der zuvor genannten Polypeptide und wobei die mindestens eine Beschichtungslage sowohl die Hyaluronsäure als auch das Polypeptid enthält

c) optionale ein- oder mehrmalige Wiederholung von Schritt b)

d) optionale Rücktrocknung der beschichteten Wundabdeckung,

Bevorzugt wird in Schritt b) zumindest die proximale Seite des Netzes beschichtet. Es können auch die proximalen Seiten von Netz und Vliesmaterial beschichtet werden, was gleichzeitig oder nacheinander geschehen kann. Es muss dabei nicht die gesamte Fläche der proximalen Seite des Vliesmaterials beschichtet werden. Eine Teilbeschichtung ist ebenfalls möglich und kann dadurch erreicht werden, dass ein Teil der durch Sprühen aufgebrachten Lösung durch die Öffnungen des Netzes hindurch auf das Vliesmaterial gelangt. Während der späteren Anwendung der Wundabdeckung kann die auf das Vliesmaterial aufgebrachte Beschichtung durch die Öffnungen des Netzes in Kontakt mit der Wunde treten.

Gemäß einer Abwandlung des obigen Herstellungsverfahrens enthaltend mindestens einen Sprühschritt wird das Netz und optional auch das Vliesmaterial allein beschichtet und anschießend mit den übrigen Komponenten der Wundabdeckung zum fertigen Produkt verbunden.

Das Besprühen kann beispielsweise mittels eines Zerstäubers oder einer Sprühpistole geschehen. Bevorzugt handelt es sich um ein elektrisches Sprühgerät, das eine Konstante Menge an Flüssigkeitsvolumen pro Zeiteinheit versprüht.

Einer der großen Vorteile dieses Verfahrens ist die Zeitersparnis. In diesem Sinne kann vorgesehen sein, dass das Aufsprühen einer Lage maximal 5 min, bevorzugt maximal 1 min, besonders bevorzugt maximal 30 s und ganz besonders bevorzugt maximal 5 s dauert. Diese Werte können bezogen sein auf ein Netz mit einer Fläche von 10cm x 10cm.

Weiterhin kann vorgesehen sein, dass der gesamte Beschichtungsprozess mittels Aufsprühen vom Beginn des Sprühvorgangs bis zum Erhalt der fertigen Wundabdeckung maximal 30 min dauert, wobei die Trocknung der Wundabdeckung in diesem Zeitraum bereits inkludiert sein kann. Diese Werte können bezogen sein auf eine Wundabdeckung mit einer Fläche von 10cm x 10cm.

Bevorzugt liegen die Hyaluronsäure und das Polypeptid oder die Polypeptide während des Besprühens gemäß Schritt ii) des obigen Herstellungsverfahrens in Lösung vor. Mögliche Lösungen wie polare Flüssigkeiten, insbesondere Wasser und wässrige Pufferlösungen werden hierin beschrieben und können im Rahmen von Herstellungsverfahren eingesetzt werden.

Bevorzugt beträgt die Konzentration des in Lösung vorliegenden Polypeptids oder der Polypeptide während des Besprühens gemäß Schritt b) bei 0,1 mg / ml bis 100 mg / ml, besonders bevorzugt bei 1 mg / ml bis 80 mg / ml, ganz besonders bevorzugt bei 3 mg / ml bis 50 mg / ml und am besten 5 mg / ml bis 30 mg / ml.

Bevorzugt beträgt die Konzentration der Hyaluronsäure während des Besprühens gemäß Schritt ii) bei 0,1 mg / ml bis 10 mg / ml, besonders bevorzugt 0,5 mg / ml bis 8 mg /ml, ganz besonders bevorzugt bei 1 mg / ml bis 5 mg / ml und am besten 2 mg / ml bis 4 mg / ml.

Bevorzugt werden durch das Besprühen gemäß Schritt b) 0,1 bis 1 ml einer solchen Lösung des Polypeptids bzw. der Polypeptide und / oder 0,1 ml bis 1 ml einer solchen Lösung der Hyaluronsäure pro cm2 der proximalen (wundzugewandten) Seite des Netzes und optional auch des Vliesmaterials aufgesprüht. Besonders bevorzugt werden 0,2 bis 0,9 ml, ganz besonders bevorzugt 0,3 bis 0,8 ml und am besten 0,4 bis 0,7 ml einer solchen Lösung aufgesprüht. Die Volumenangaben sind pro Lage zu verstehen. Da im Falle durchmischter Lagen bereits eine einzige Lage eine fertige Beschichtung ausbilden kann, können die Volumenangaben in einem solchen Fall auch pro Beschichtung (enthaltend eine Lage) verstanden werden.

Gemäß einem weiteren Aspekt der Erfindung wird bei dem oben beschriebenen Verfahren das Besprühen gemäß Schritt b) mindestens zwei Mal ausgeführt, um eine Beschichtung mit mindestens zwei Lagen zu erzeugen. Zwischen dem Besprühen kann eine Trocknungsphase liegen. Diese kann 30 s bis 30 min betragen, bevorzugt 1 min bis 25 min, ganz besonders bevorzugt 2 min bis 20 min und am besten 3 min bis 15 min.

Teil der Erfindung ist auch eine Wundabdeckung wie hierin beschrieben, erhältlich oder erhalten durch das zuletzt erläuterte Herstellungsverfahren.

Ein anderes erfindungsgemäßes Verfahren zur Herstellung einer beschichteten Wundabdeckung umfasst die folgenden Schritte:

a) Bereitstellung einer Wundabdeckung umfassend ein Kunststoffnetz und ein absorbierendes Vliesmaterial, wobei das Netz auf seiner von der Wunde abgewandten Seite von dem Vliesmaterial überlagert wird,

b) Beschichten des Netzes durch Auftragen von mindestens zwei übereinanderliegenden Lagen, wobei mindestens eine Lage i) eine Hyaluronsäure enthält und eine negative Nettoladung hat und mindestens eine Lage ii) ein Polypeptid enthält und eine positive Nettoladung hat, wobei das Polypeptid ausgewählt ist aus Polyarginin, Polylysin und Polyornithin oder einer Mischung aus mindestens zwei der zuvor genannten Polypeptide und mindestens eine Lage Hyaluronsäure enthält und wobei die übereinanderliegenden Lagen in ihrer Nettoladung alternieren

c) optionale ein- oder mehrmalige Wiederholung von Schritt ii)

d) optionales Rücktrocknen der beschichteten Wundabdeckung.

Bevorzugt wird in Schritt ii) zumindest die proximale Seite des Netzes beschichtet. Alternativ können in Schritt ii) zumindest die proximalen Seiten von Netz und Vliesmaterial beschichtet werden. Die Beschichtung kann Teile der proximale Seite bedecken oder vollflächig auf der proximalen Seite ausgebildet werden.

Gemäß einer Abwandlung des obigen Herstellungsverfahrens mittels Einsatz von Tauchbeschichtung werden das Netz und optional auch das Vliesmaterial allein beschichtet und anschießend mit den übrigen Komponenten der Wundabdeckung zum fertigen Produkt verbunden.

Der Beschichtungsschritt b) kann insbesondere mittels Tauchverfahren ausgeführt werden. Andere Beschichtungstechniken sind ebenfalls möglich. So kann das Beschichten beispielsweise alternativ auch durch Auftragung mittels Walze oder durch Bestreichen (z.B. mittels Pinsel) erfolgen.

Die negative Nettoladung der Hyaluronsäure enthaltenden Lage wird durch die negativ geladene Hyaluronsäure vermittelt und die positive Nettoladung der Polypeptid enthaltenden Lage wird durch die positiv geladenen Aminosäuren des Polypeptids vermittelt.

Empfohlen wird sowohl die Hyaluronsäure als auch das Polypeptid oder die Polypeptide vor dem Beschichten in Lösung zu bringen. Geeignete Lösungsmittel und Konzentrationen wurden hierin bereits an anderer Stelle beschrieben und sind im Rahmen des zuletzt genannten Verfahrens anwendbar. So können das Polypeptid oder die Polypeptide beispielsweise mittels einer Lösung aufgebracht werden, in der sie in einer Konzentration von 0,1 mg / mL bis 100 mg / mL enthalten sind und / oder beispielsweise die Hyaluronsäure mittels einer Lösung aufgetragen werden, die die Hyaluronsäure in einer Konzentration von 0,1 bis 10 mg / mL enthält.

Dieses Verfahren eignet sich insbesondere zur Herstellung solcher Wundabdeckungen, deren Beschichtung mindestens zwei nicht durchmischte Lagen enthält. Nicht durchmischte Lagen sind an anderer Stelle hierin in ihrer Beschaffenheit erläutert. In der Regel enthalten die mit dem obigen Herstellungsverfahren erzeugten Beschichtungen mindestens zwei Lagen. Bevorzugt sind sämtliche Lagen der mittels dieses Verfahrens erzeugten Beschichtung nicht durchmischt. Dabei können sämtliche Lagen mittels Tauchbeschichtung erzeugt werden.

Im Rahmen des zuletzt genannten Herstellungsverfahrens ist es möglich mittels Tauchbeschichtung eine Vielzahl von Lagen zu einer Beschichtung zu vereinen. Empfohlen wird im Rahmen von Schritt b) nach dem Auftragen einer Lage diese mit einer Pufferlösung zu spülen. Dies sollte erst dann geschehen, wenn die zu spülende Lage ausreichend getrocknet oder angetrocknet ist, um ein ungewolltes Abwaschen zu vermeiden.

Das (vollständige oder teilweise) Eintauchen kann dabei für die Erzeugung einer einzelnen Lage über eine Zeitspanne von ein bis zehn Minuten erfolgen. Das heißt, dass Netz, Netz und Vliesmaterial oder die Wundabdeckung als Ganzes in die Lösung eingetaucht wird und nach ein bis zehn Minuten entnommen wird. Die folgenden alternativen Zeitspannen sind ebenfalls möglich: zwei bis neun Minuten, drei bis acht Minuten sowie vier bis sieben Minuten.

Teil des zuletzt genannten Verfahrens ist darüber hinaus, dass Schritt b) wiederholt stattfinden kann (also mindestens zwei Mal). So kann Schritt b) für die Herstellung einer erfindungsgemäßen Wundabdeckung beispielsweise zehn bis hundert Mal wiederholt werden, so dass zehn bis hundert Lagen übereinanderliegend aufgetragen werden. Dies resultiert folglich in einer Wundabdeckung mit einer Beschichtung, die zehn bis hundert Lagen enthält. Andere mögliche Wiederholungsanzahlen für Schritt b) sind 15 bis 90 Mal, 20 bis 80 Mal, 25 bis 70 Mal und 30 bis 60 Mal.

Wenn die Beschichtungsauftragung gemäß Schritt b) mittels Tauchverfahren ausgeführt wird, beziehen sich die eben genannten Wiederholungszahlen auf die Anzahl des Eintauchens in eine Lösung enthaltend die Hyaluronsäure oder das Polypeptid.

Teil der Erfindung ist auch eine beschichtete Wundabdeckung wie hierin beschrieben, erhältlich oder erhalten durch das zuletzt erläuterte Herstellungsverfahren unter Einsatz von Tauchbeschichtung.

Die hierin beschriebenen Herstellungsverfahren können sich bei Bedarf durch folgende Punkte auszeichnen:

- Es kann eine Seite oder es können beide Seiten des Netzes (proximale und distale Oberfläche) beschichtet werden. Wenn beide Seiten beschichtet werden, so kann dies nacheinander oder gleichzeitig erfolgen.

- Auf ein Netz mit negativer Nettoladung oder negativer Außenladung (negative Ladung der Oberfläche) kann zuerst eine Lage enthaltend das Polypeptid als erste Lage aufgetragen werden. In diesem Fall hat die erste Lage eine positive Nettoladung. Bevorzugt enthält in diesem Fall die erste Lage keine Hyaluronsäure.

- Auf ein Netz mit positiver Nettoladung oder positiver Außenladung (positive Ladung der Oberfläche) kann zunächst eine Lage enthaltend die Hyaluronsäure als erste Lage aufgetragen werden. In diesem Fall hat die erste Lage eine negative Nettoladung. Bevorzugt enthält in diesem Fall die erste Lage kein Polypeptid bzw. keine Polypeptide.

Ein weiterer Teil der Erfindung betrifft die hierin beschriebenen Herstellungsverfahren, wobei die bereitgestellte Wundabdeckung oder deren Netz zunächst einer Plasmareinigung unterzogen wird. Die Plasmareinigung findet vor der Beschichtung, Auftragung oder dem Besprühen statt und erlaubt ein noch besseres Anhaften der späteren Beschichtung auf dem Netz und optional auch auf dem Vliesmaterial.

Weiterhin können die hierin beschriebenen Herstellungsverfahren automatisiert sein. Bevorzugt findet die Automatisierung mittels eines Roboters statt. Bei dem Roboter kann es sich um einen programmierbaren Roboter handeln. Der Roboter kann über mindestens einen beweglichen Arm verfügen, an dem ein oder mehrere Netze (optional zusammen mit dem Vliesmaterial) oder aber ein oder mehrere Wundabdeckungen angebracht werden können. Insbesondere das Auftragen der Beschichtung mittels Tauchverfahren profitiert von einer Automatisierung, da diese Methode in der Regel mehr Zeit beansprucht als das Auftragen der Beschichtung mittels Sprühverfahren. Bevorzugt werden bei dem automatisierten Tauchverfahren Beschichtungen mit mindestens 20, besser 25 und am besten 30 Lagen auf dem Netz und optional auch auf dem Vliesmaterial erzeugt. Wenn das erfindungsgemäße Herstellungsverfahren mittels eines Roboters stattfindet, können mehrere Netze und optional auch Vliesmaterialien gleichzeitig beschichtet werden. Beispielsweise können zwei, mindestens zwei, drei, mindestens drei, vier oder mindestens vier, fünf oder mindestens fünf Netze und / oder Vliesmaterialien gleichzeitig auf diese Weise beschichtet werden. Besonders bevorzugt werden zwei bis hundert, zwei bis fünfzig oder zwei bis zehn Netze und optional auch Vliesmaterialien gleichzeitig unter Einsatz eines einzigen Roboters zeitgleich beschichtet.

Das Sprühverfahren profitiert ebenfalls von einer Automatisierung, da hierdurch ein gleichbleibender Abstand zwischen Sprühdüse und Netz und optional auch Vliesmaterial während des Sprühvorgangs gewährleistet wird, was bei einem händischen Ansatz deutlich schwieriger umzusetzen ist.

Daneben betrifft die Erfindung auch die Verwendung der erfindungsgemäßen Wundabdeckung als Wundkontaktschicht.

Ein weiterer Aspekt der Erfindung betrifft die erfindungsgemäße Wundabdeckung zur Anwendung in einem Verfahren zur Behandlung von Wunden, bevorzugt von infizierten Wunden, besonders bevorzugt von Wunden, die mit S. aureus und / oder P. aeruginosa infiziert sind.

Ein weiterer Aspekt der Erfindung betrifft die erfindungsgemäße Wundabdeckung zur Anwendung in einem Verfahren zur Behandlung von Wunden, bevorzugt von infizierten Wunden, wobei die Beschichtung auf einem Netz - wie hierin beschrieben - vorliegt.

Bei den beiden zuletzt genannten Anwendungsfällen kann vorgesehen sein, dass die Anwendung über einen Zeitraum von 0 bis 24 Stunden stattfindet oder, dass die Anwendung in einem Zeitraum von mindestens 24 Stunden stattfindet, wobei letzteres beispielsweise bei bereits stark ausgeprägten Infektionen oder bei Patienten mit Immunschwäche geboten sein kann.

Die erfindungsgemäße Wundabdeckung ist geeignet zur Abdeckung und / oder Therapie von Wunden, insbesondere infizierten Wunden. Aus diesen Gründen kann die Wundabdeckung eingesetzt oder verwendet werden in einem Verfahren zur Wundtherapie und / oder zur Reduktion der Keimzahl in Wunden und / oder zur Prävention von Wundinfektionen. Bei den Wunden kann es sich insbesondere um traumatische Wunden (inklusive Schnitt- und Operationswunden), chronische Wunden, blutende Wunden, eiternde Wunden und exsudierende (nässende) Wunden handeln. Die erfindungsgemäße Wundabdeckung kann auch im Rahmen der Kompressionstherapie unter Kompressionsbandagen oder Kompressionsstrümpfen getragen werden.

Figuren

Im Folgenden werden die Figuren näher erläutert. Wo Keimzahlen dargestellt sind, erfolgt diese Darstellung in den meisten Fällen anhand eines dekadischlogarithmischen Maßstabs der Ordinate.

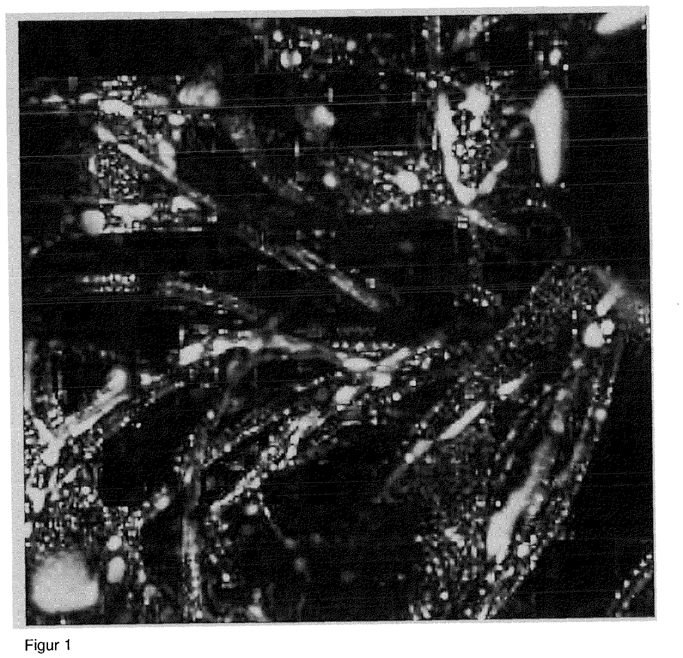

Fig. 1 zeigt eine Fluoreszenzaufnahme eines tauchbeschichteten Kunststoffnetzes inklusive absorbierendem Vliesmaterial mittels Konfokalmikroskop. Die Beschichtung enthält 24 Doppellagen aus Polyarginin und Hyaluronsäure, wobei das in der Beschichtung enthaltene Polyarginin mittels des fluoreszierenden Markers FITC sichtbar gemacht wurde.

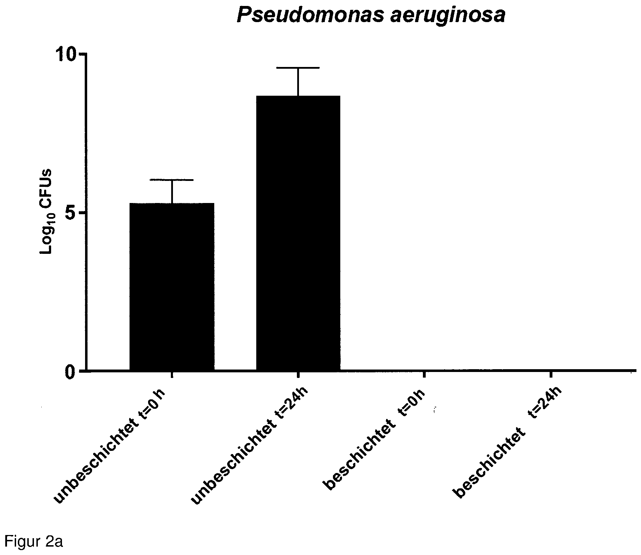

Fig. 2a zeigt die antimikrobielle Wirksamkeit von Kunststoffnetzen inklusive absorbierendem Vliesmaterial, wobei Netz und Vlies mit einer polyargininhaltigen Tauchbeschichtung (48 alternierende Einzelschichten aus entweder Polyarginin oder Hyaluronsäure; entsprechend 24 Doppelschichten) ausgestattet wurden, gegenüber P. aeruginosa nach Initialkontakt (t = 0h) sowie einen Tag später (t = 24 h). Der Test erfolgte nach ISO 20743:2021.

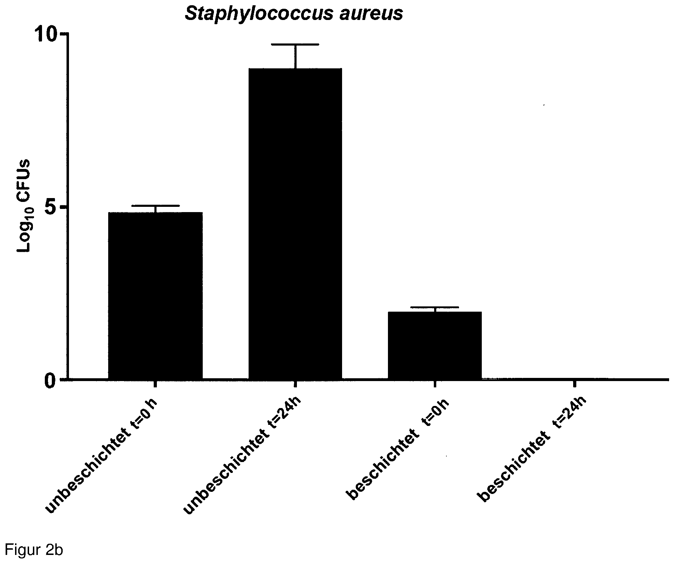

Fig. 2b zeigt Werte für die gleiche Anordnung wie für Fig. 2a beschrieben, allerdings in diesem Fall gegenüber S. aureus.

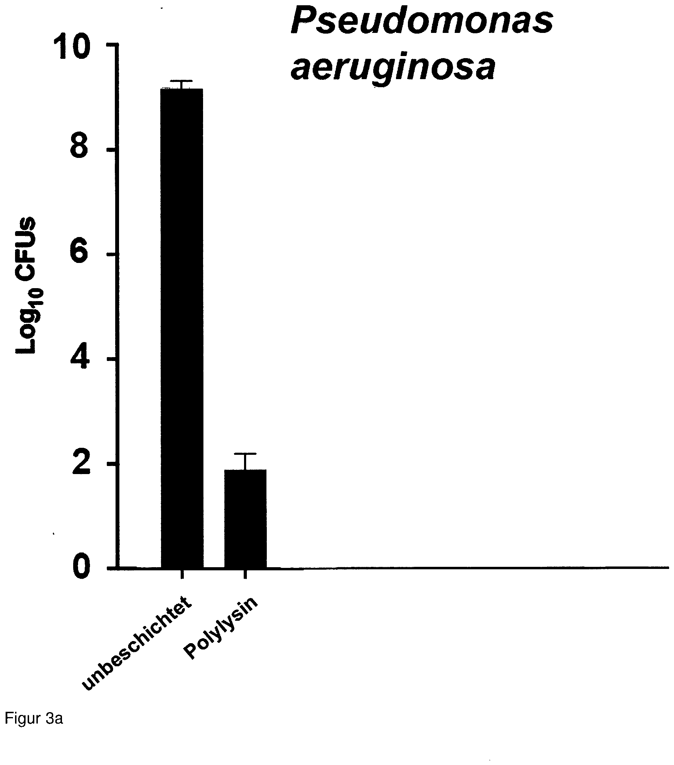

Fig. 3a zeigt die antimikrobielle Wirksamkeit von Kunststoffnetzen inklusive absorbierendem Vliesmaterial, wobei Netz und Vlies mit einer polylysinhaltigen Sprühbeschichtung (eine durchmischte Lage enthaltend Polylysin und Hyaluronsäure) ausgestattet wurden, gegenüber P. aeruginosa nach einer Wirkungsdauer von einem Tag. Dargestellt sind die Mittelwerte aus drei Wiederholungen. Der Test erfolgte nach ISO 20743:2021.

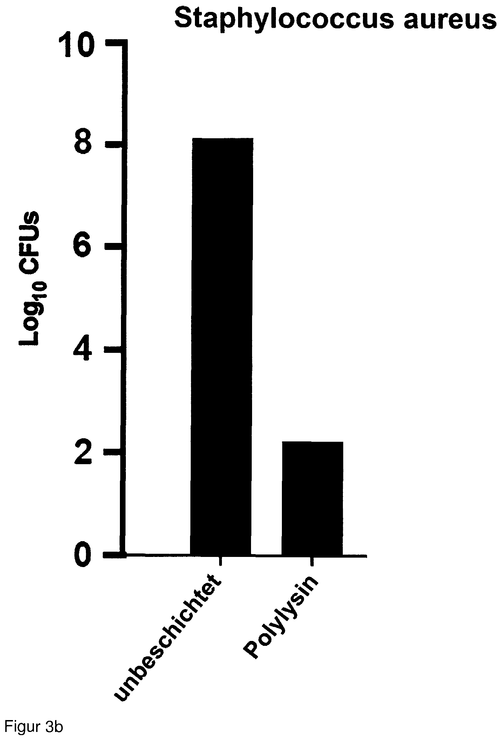

Fig. 3b zeigt Werte für die gleiche Anordnung wie für Fig. 3a beschrieben, allerdings in diesem Fall gegenüber S. aureus.

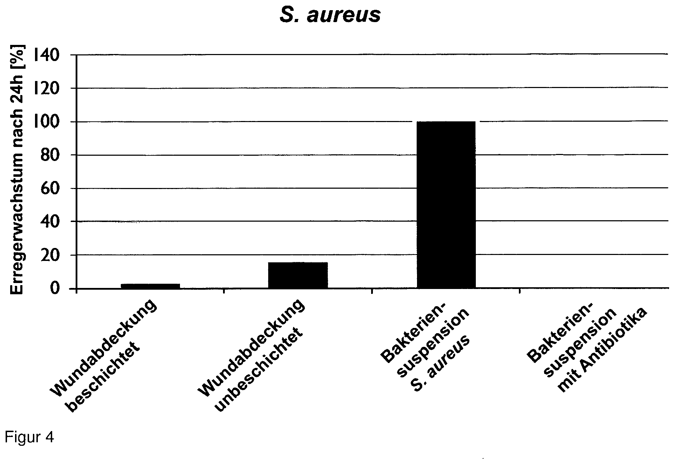

Fig. 4 zeigt das Wachstum von *S. aureus* nach einer Kontaktzeit der Bakteriensuspension von T= 24 h mit einer tauchbeschichteten Wundabdeckung, deren antimikrobielle Beschichtung aus 24 Doppellagen bestand, wobei jede Doppellage aus einer Lage enthaltend Polyarginin und einer Lage enthaltend Hyaluronsäure bestand (48 Einzellagen). Negativkontrolle = unbeschichtete baugleiche Wundabdeckung; Positivkontrolle = Bakteriensuspension versetzt mit Antibiotika; *S. aureus* = Wachstum in der bakteriellen Suspension ohne Wundabdeckung oder Antibiotika ; die Ordinate gibt das bakterielle Wachstum als prozentuale Angabe wieder.

**Beispiele**

**Beispiel 1: Materialien**

An Materialien wurden bereitgestellt:

- Polypeptide vom Typ Polyarginin als synthetisches Polymer bestehend aus 30 Aminosäuren pro Molekül ("PAR30"), so dass jedes Molekül eine Molekülmasse von etwa 5,8 kDa hatte. Das Polyarginin wurde vom Unternehmen "AlamandaTM Polymers" bezogen. Die eingesetzte Konzentration lag bei 0,5 mg / ml.

- Polypeptide vom Typ Polylysin, welche als $\varepsilon$-Poly(L-Lysin) natürlichen Ursprungs (Herstellung mittels Bakterien aus der Familie der Streptomycetaceae) vom Unternehmen Biosynth® bezogen wurden. Die mittlere Molekülmasse lag zwischen 3,5 und 4,5 kDa. Die eingesetzte Konzentration lag bei 10 mg / ml Tris-NaCl-Puffer.

- Hyaluronsäure aus 144 repetitiven Untereinheiten pro Molekül ("HA144"). Hergestellt mittels rekombinanter Produktion aus Mikroorganismen. Die eingesetzte Konzentration lag bei 0,5 mg / ml Tris-NaCl-Puffer. Bezogen wurde die Hyaluronsäure vom Unternehmen Lifecore® Biomedical.

- An Träger wurde bereitgestellt:

    Kunststoffnetz: Extrudierter Polyethylenfilm, der über einen Perforationsroller geführt und anschließend zur Einstellung der Perforationsgröße biaxial gedehnt wurde. Das Netz war überlagert von und verbunden mit einem absorbierenden Vliesmaterial.
    Absorbierendes Vliesmaterial: Spinnvliesstoff aus 10 Gew.-% Polyethylen-Polypropylen-Gemisch und 90 Gew.-% Viskose.
    Der resultierende Träger hatte eine Fläche von 5,5 cm x 5,5 cm, Dicke von 1,35 mm, ein Flächengewicht von 127 g/m$^2$ und eine Absorptionskapazität von über 900 Gew.-% seines Eigengewichts.

**Beispiel 2: Beschichtung mittels Tauchverfahren**

Als Ausgangsmaterial diente der Träger wie unter Beispiel 1 beschrieben.
Der Träger wurde auf eine Fläche von 2 cm x 2 cm zugeschnitten und zunächst in einem Autoklaven sterilisiert. Während des nachfolgenden Beschichtungsvorgangs mittels Tauchverfahren wurde der Träger abwechselnd in ein Bad mit Polyarginin ("PAR30" ; 0,5 mg / mL) und ein Bad mit Hyaluronsäure (0,5 mg / mL) eingetaucht. Der erste Eintauchschritt erfolgte dabei in der Lösung mit Polyarginin. Das Eintauchen erfolgte jeweils für 200 Sekunden. Nach jedem Tauchschritt wurde der Träger gespült, und zwar ebenfalls für 200 Sekunden. Hierzu wurde Tris-NaCl-Puffer verwendet (10 mmol Tris, 150 mmol NaCl, pH 7,4). Der Prozess fand voll automatisiert statt unter Einsatz eines Roboters der Firma Riegler & Kirstein GmbH.

Der Programmablauf des Roboters sah wie folgt aus:

$$A6+(\underline{A5}+A6+B5+B6+\mathit{A4}+A3+B4+B3)*8+(\underline{A5}+A6+A7+B7+\mathit{A4}+A3+A2+B2)$$
$$*8+(\underline{A5}+A6+A8+B8+\mathit{A4}+A3+A1+B1)*8+eB1$$

Legende:

A, B = Positionen des Roboterarms
unterstrichen = Auftrag des Polypeptids
kursiv = Auftrag der Hyaluronsäure
Programmdauer: ca. 13h

Die genannten Schritte wurden so oft wiederholt, bis 24 Doppellagen auf den Träger aufgetragen waren. Jede Doppellage bestand aus einer Lage enthaltend das Polyarginin und einer Lage enthaltend die Hyaluronsäure. Somit enthielt die fertige Beschichtung 48 Einzellagen. Der Beschichtungsvorgang dauerte insgesamt etwa 13 Stunden. Bei Bedarf kann der Prozess auch auf eine Dauer von unter zwei Stunden verkürzt werden. Nach einer Trocknung (über Nacht, passiv bei Raumtemperatur) erfolgte im Anschluss eine weitere Sterilisation mittels U.V.-Bestrahlung, und zwar über einen Zeitraum von mindestens 30 min für jeweils die proximale und distale Oberfläche der Träger.

**Beispiel 3: Auftrag einer polylysinhaltigen Beschichtung mittels Sprühverfahren**

Als Ausgangsmaterial diente der Träger wie unter Beispiel 1 beschrieben. Es wurden vier Prüfmuster mit einer Fläche von jeweils 2,25 $cm^2$ bereitgestellt. Bei der Beschichtung mittels Sprühverfahren kamen eine Lösung enthaltend ε-Poly(L-Lysin) (10 mg / ml) und eine Lösung enthaltend Hyaluronsäure ("HA144" ; 0,5 mg / ml) zum Einsatz. Als Lösungsmittel diente Tris-NaCl-Puffer. Beide Lösungen wurden zeitgleich über jeweils eine separate Düse einer Sprühpistole auf die Netzseite (proximale Seite) des Trägers aufgesprüht. Das Aufsprühen fand aus einer Distanz von 10 bis 15 cm statt. Dabei wurde die Sprühpistole während des Sprühens zehn Mal für jeweils eine Sekunde über jedes Prüfmuster geführt, um pro Muster insgesamt 1,5 ml von jeder Lösung aufzutragen. Somit Betrug das Flächenvolumen der Beschichtungslösung 0,66 ml / $cm^2$ Trägeroberfläche. Der Beschichtungsvorgang dauerte 10 - 15 Sekunden. Anschließend folgte eine Trocknung (über Nacht, passiv bei Raumtemperatur).

**Beispiel 4: Visuelle Inspektion der Tauchbeschichtung mittels Konfokalmikroskop**

Die im Tauchverfahren gemäß Beispiel 2 erzeugte Beschichtung wurde einer visuellen Prüfung mittels Konfokalmikroskop unterzogen. Hierzu wurde das in der Beschichtung enthaltene Polyarginin mit dem Fluorophor Fluoresceinisothiocyanat (FITC) markiert (PAR30-FITC).

Die Verteilung der auf diese Weise zur Fluoreszenz gebrachten Moleküle wurde mit einem Mikroskop vom Typ Zeiss LSM 710 (Konfokales Laserscanning Mikroskop) begutachtet. Dies fand unter einer 40-fachen Vergrößerung statt. Die korrekte Anhaftung der Beschichtung an der Faserstruktur des verwendeten Vliesmaterials konnte verifiziert werden. Das Ergebnis ist in Fig. 1 als kontrastverstärkte Fotoaufnahme dargestellt.

Wie zu erkennen ist, umschließt die Beschichtung die Faserstruktur des Vliesmaterials zu allen Seiten auf gleichmäßige Weise. Dies lässt auf eine erfolgreiche Anhaftung der Beschichtung schließen. Eine unbeschichtete Kontrollaufnahme eines ansonsten identischen Trägers zeigte keinerlei Fluoreszenz (nicht abgebildet).

**Beispiel 5: Antimikrobielle Wirksamkeit einer polyargininhaltigen Tauchbeschichtung**

Die Tests zur antimikrobiellen Wirksamkeit wurden an den in Beispiel 2 mittels Tauchverfahren erzeugten beschichteten Trägern durchgeführt, wobei sowohl gramnegative als auch grampositive Bakterienstämme zum Einsatz kamen. Als gramnegative Kultur diente ein Stamm von *Pseudomonas aeruginosa* (aus Hinterlegung ATTC 27853) und als grampositive Kultur ein Stamm von *Staphylococcus aureus* (aus Hinterlegung ATTC 25923).

Als Testprotokoll diente der Standard ISO 20743:2021 (Owen L, Laird K. Development of a silver-based dual-function antimicrobial laundry additive and textile coating for the decontamination of healthcare laundry. J Appl Microbiol. 2021 ;130(4):1012-22). Beim Test wurde die Reduktion der Anzahl teilungsfähiger Bakterienzellen (CFU) auf den textilen Oberflächen nach einer Kontaktzeit von 0 h und 24 h ermittelt. Die antimikrobielle Beschichtung war zuvor im Tauchverfahren auf die Textilien aufgetragen worden und bestand aus einer alternierenden Abfolge von PAR30 und HA144 mit 48 Einzellagen. Weitere Details können Beispiel 2 entnommen werden.

Zunächst wurden die beschichteten Träger mit einer Bakterienausgangskonzentration von 1 bis 3 x $10^5$ CFU / ml beimpft und bei 37 °C inkubiert. Im Anschluss (nach 0 bzw. 24 h) wurden die überlebenden Bakterien in PBS eluiert. Das Eluat wurde abwechselnd gevortext (mit hoher Frequenz mittels eines Vortex Gerätes geschüttelt), sonifiziert und wieder gevortext (jeweils 30 Sek. in dreifacher Wiederholung). Die Proben wurden sowohl im trockenen als auch im feuchten Zustand (nach Zugabe von PBS) getestet, um ein feuchtes Wundmilieu zu simulieren. Als Kontrolle dienten unbeschichtete Träger mit ansonsten identischem Aufbau. Die Tests wurden mit drei biologischen und drei technischen Wiederholungen durchgeführt. Die ermittelte antimikrobielle Aktivität ist ausgedrückt als Mittelwert der logarithmischen Reduktion der Anzahl vermehrungsfähiger Bakterien im Vergleich zur Kontrolle. Die Auswertung der Ergebnisse zeigte eine Keimreduktion von ≥ 8 log10 für *P. aeruginosa* und ≥ 8 log10 für S. *aureus.* Die Ergebnisse sind dargestellt in Fig. 2a (*P. aeruginosa*) und Fig. 2b (*S. aureus*). Wie aus der Darstellung deutlich wird, setzte bereits nach Initialkontakt mit der Beschichtung (0 h) eine starke antimikrobielle Wirkung ein, deren Effekt nach 24 h noch ausgeprägter war. Die Befeuchtung der Träger mit PBS hatte keinen Einfluss auf die Ergebnisse (nicht dargestellt).

**Beispiel 6: Antimikrobielle Wirksamkeit einer polylysinhaltigen Sprühbeschichtung**

Für den Test eingesetzt wurden die in Beispiel 3a mittels Sprühverfahren beschichteten Träger. Die Träger waren ausgestattet mit einer Sprühbeschichtung bestehend aus einer durchmischten Lage enthaltend ε-Poly(L-Lysin) (10 mg / ml) und HA144 (0.5mg / ml). Die antimikrobielle Wirksamkeit der mittels Spühbeschichtung ausgestatteten Träger wurde mittels ISO 20743:2021 getestet. Die Messung wurde insgesamt drei Mal durchgeführt und die Mittelwerte gebildet. Weitere Details zum Ablauf dieses Tests können Beispiel 5 entnommen werden, wobei im vorliegenden Fall keine Messwerte für den Initialkontakt (t = 0) genommen wurden. Fig. 3a und Fig. 3b zeigen die nach 24 h erhobenen Messwerte. Wie aus den Darstellungen deutlich wird, war ein ausgeprägter antimikrobieller Effekt nachweisbar.

Die Auswertung der Ergebnisse zeigte eine Keimreduktion von ≥ 7 log10 für P. *aeruginosa* und ≥ 6 log10 für S. *aureus.*

**Beispiel 7: Trocknung von Trägern mit aufgesprühter Beschichtung**

Die im oben beschriebenen Beispiel 3 hergestellten sprühbeschichteten Träger wurden nach dem Beschichtungsprozess über Nacht bei Raumtemperatur getrocknet.

Zusätzlich fanden antimikrobielle Versuche mit sprühbeschichteten, noch feuchten Proben sowie mit sprühbeschichteten Proben, die 10 min lang bei 80 °C getrocknet wurden, statt. Die Sprühbeschichtung wurde auch hier gemäß Beispiel 3 aufgetragen.

Die ermittelte antimikrobielle Wirkung war in allen Fällen nahezu identisch (Ergebnisse nicht dargestellt), so dass geschlussfolgert werden kann, dass die antiseptische Wirksamkeit auch bei unterschiedlichem Feuchtigkeitsgehalt der Beschichtung sowie nach aktiver Trocknung erhalten bleibt.

**Beispiel 8: Antimikrobielle Wundauflage enthaltend Kunststoffnetz**

Bereitgestellt wurde eine Wundauflage mit einer Gesamtfläche von 10 cm x 10 cm. Proximal war der in Beispiel 1 erläuterte Träger angebracht, bestehend aus dem wundseitig gelegenen Kunststoffnetz, welches überlagert war von dem absorbierenden Vliesmaterial. Oberhalb (distal) vom Vliesmaterial war ein mit synthetischem Acrylatkleber beschichtetes Backing aus dünnem Vlies (100 % Polyester) aufgeklebt. Das Backing bildete einen über Netz und Vliesmaterial überstehenden umlaufenden Kleberand, so dass die Wundauflage als Inselverband ausgestaltet war.

Die derart beschaffene Wundauflage wurde mithilfe des in Beispiel 2 erläuterten Tauchbeschichtungsverfahrens mit einer antimikrobiellen Beschichtung ausgestattet. Die Beschichtung bedeckte sowohl Netz als auch Vliesmaterial und bestand analog zu Beispiel 2 aus 24 Doppellagen. Jede Doppellage bestand aus einer Lage enthaltend das Polyarginin und einer Lage enthaltend die Hyaluronsäure. Somit enthielt die fertige Beschichtung 48 Einzellagen.

In der Folge wurde die antimikrobielle Aktivität gegenüber S. *aureus* getestet. Als Negativkontrolle diente eine baugleiche Wundauflage ohne Beschichtung. Für die Positivkontrolle wurde der Bakteriensuspension Antibiotikum zugefügt.

Als Protokoll diente eine vereinfachter, interner Teststandard, der weitestgehend ähnlich zu den offiziellen Protollen ISO20743 und AATCC100 ablief. Bestimmt wurde das Bakterienwachstum im Überstand nach 24 h Kontaktzeit mit der antimikrobiellen Beschichtung. Die Ergebnisse sind in Fig. 4 dargestellt. Die Säulen repräsentieren das Erregerwachstum in Relation zum Ausgangszeitpunkt.

**Patentansprüche**

1. Wundabdeckung umfassend

   - ein Kunststoffnetz,
   - ein absorbierendes Vliesmaterial,

   wobei das Netz auf seiner von der Wunde abgewandten Seite von dem Vliesmaterial überlagert wird, und wobei zumindest das Netz wundseitig eine teilweise oder vollständige antimikrobielle Beschichtung aufweist, welche i) eine Hyaluronsäure sowie ii) ein Polypeptid ausgewählt aus Polyarginin, Polylysin und Polyornithin oder eine Mischung aus mindestens zwei der zuvor genannten Polypeptide umfasst.

2. Wundabdeckung nach Anspruch 1, wobei das Polypeptid mindestens zehn Aminosäuren und/oder höchstens hundert Aminosäuren umfasst.

3. Wundabdeckung nach Anspruch 1 oder 2, wobei die Hyaluronsäure als Polymermischung mit unterschiedlicher Kettenlänge vorliegt und wobei die Polymermischung Polymere der Hyaluronsäure mit einem Molekulargewicht von mindestens 10 kDa und / oder höchstens 300 kDa umfasst.

4. Wundabdeckung nach einem der vorhergehenden Ansprüche, wobei die Beschichtung mindestens zwei übereinanderliegende und miteinander verbundenen Lagen enthält, und wobei mindestens eine Lage vorhanden ist, welche die Polypeptide enthält und eine positive Nettoladung hat, sowie mindestens eine Lage, welche die Hyaluronsäure enthält und eine negative Nettoladung hat, und wobei sich bei den übereinanderliegenden Lagen jeweils eine die Polypeptide enthaltende Lage mit einer die Hyaluronsäure enthaltenden Lage abwechselt, so dass eine Abfolge von alternierenden Lagen gebildet wird.

5. Wundabdeckung nach Anspruch 4, wobei die Anzahl der übereinanderliegenden und miteinander verbundenen Lagen in der Beschichtung 10 bis 100 beträgt.

6. Wundabdeckung nach einem der Ansprüche 1 bis 3, wobei die Polypeptide und die Hyaluronsäure innerhalb der Beschichtung oder mindestens einer Lage der Beschichtung durchmischt vorliegen und die Polypeptide in einer Hyaluronsäurematrix eingebettet sind.

7. Wundabdeckung nach einem der vorhergehenden Ansprüche, wobei die Polypeptide ein Molekulargewicht von 1 bis 41 kDa haben.

8. Wundabdeckung nach einem der vorhergehenden Ansprüche, wobei die Beschichtung eine Dicke von 10 nm bis 1000 nm hat.

9. Wundabdeckung nach einem der vorhergehenden Ansprüche, wobei das Kunststoffnetz faserlos ausgestaltet ist und / oder aus einem perforierten Film besteht.

10. Wundabdeckung nach einem der vorhergehenden Ansprüche, wobei das Kunststoffnetz Polyethylen enthält.

11. Wundabdeckung nach einem der vorhergehenden Ansprüche, wobei das absorbierende Vliesmaterial mindestens zwei verschiedene Faserarten enthält.

12. Wundabdeckung nach einem der vorhergehenden Ansprüche, wobei das absorbierende Vliesmaterial Fasern aus Viskose, Polyethylen und entweder

    a) Polypropylen oder
    b) Polyethylenterephthalat

    enthält und mindestens 80 Gew.-% des absorbierenden Vliesmaterials aus Viskose bestehen.

13. Wundabdeckung nach einem der vorhergehenden Ansprüche mit einem Flächengewicht von 100 g/m$^2$ bis 180 g/m$^2$ und / oder einer Dicke von 1 bis 5 mm.

**14.** Wundabdeckung nach einem der vorhergehenden Ansprüche umfassend als distale Abschlussschicht ein Backing, welches einen um das Vliesmaterial umlaufenden Rand ausbildet, wobei dieser Rand wundseitig zumindest teilweise mit einem hautkompatiblen Adhäsiv beschichtet ist.

**15.** Wundabdeckung nach Anspruch 14 enthaltend zwischen dem Vliesmaterial und dem Backing eine Retentionsschicht mit superabsorbierenden Fasern, welche quervernetztes Acrylat-Copolymer umfassen, wobei die superabsorbierenden Fasern

> a) 15 bis 30 Gew.-% der Retentionsschicht oder
> b) 2 bis 6 Gew.-% des Faseranteils von Vliesmaterial und Retentionsschicht ausmachen.

**16.** Verfahren zur Herstellung einer Wundabdeckung nach einem der Ansprüche 6 bis 15, welche eine antimikrobielle Beschichtung enthält, wobei das Verfahren die folgenden Schritte umfasst:

> a) Bereitstellen einer Wundabdeckung umfassend ein Kunststoffnetz und ein absorbierendes Vliesmaterial , wobei das Netz auf seiner von der Wunde abgewandten Seite von dem Vliesmaterial überlagert wird,
> b) Besprühen zumindest des Netzes aus Kunstfasern mit i) einer Hyaluronsäure und ii) einem Polypeptid zur Ausbildung einer Beschichtung umfassend mindestens eine Beschichtungslage, wobei das Polypeptid ausgewählt ist aus Polyarginin, Polylysin und Polyornithin oder einer Mischung aus mindestens zwei der zuvor genannten Polypeptide und wobei die mindestens eine Beschichtungslage sowohl die Hyaluronsäure als auch das Polypeptid enthält.

Figur 1

Figur 2a

**Staphylococcus aureus**

Figur 2b

Figur 3a

Figur 3b

S. aureus

Figur 4

Europäisches
Patentamt

European
Patent Office

Office européen
des brevets

**EUROPÄISCHER RECHERCHENBERICHT**

Nummer der Anmeldung

EP 24 31 5420

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2022/211914 A1 (LAVALLE PHILIPPE [FR] ET AL) 7. Juli 2022 (2022-07-07) <br> * Seite 2, Absatz 30 * <br> * Seite 8, Absatz 161 * <br> * Seite 12, Absatz 252 * <br> * Beispiel 5 * <br> * Ansprüche 1, 2 * <br> ----- | 1-16 | INV. <br> A61L15/22 <br> A61L15/46 |

**RECHERCHIERTE SACHGEBIETE (IPC)**

A61L

Der vorliegende Recherchenbericht wurde für alle Patentansprüche erstellt

| Recherchenort | Abschlußdatum der Recherche | Prüfer |
|---|---|---|
| Den Haag | 19. Februar 2025 | Heck, Georg |

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 24 31 5420

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-02-2025

| Im Recherchenbericht angeführtes Patentdokument | Datum der Veröffentlichung | Mitglied(er) der Patentfamilie | Datum der Veröffentlichung |
|---|---|---|---|
| US 2022211914 A1 | 07-07-2022 | CA 3135244 A1 | 05-11-2020 |
| | | CN 114040785 A | 11-02-2022 |
| | | EP 3797800 A1 | 31-03-2021 |
| | | EP 3962543 A1 | 09-03-2022 |
| | | ES 2981338 T3 | 08-10-2024 |
| | | JP 7587524 B2 | 20-11-2024 |
| | | JP 2022531769 A | 11-07-2022 |
| | | US 2022211914 A1 | 07-07-2022 |
| | | WO 2020221896 A1 | 05-11-2020 |

Für nähere Einzelheiten zu diesem Anhang : siehe Amtsblatt des Europäischen Patentamts, Nr.12/82

EPO FORM P0461

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1755569 B9
- EP 3452118 B1
- EP 2371335 B1
- WO 2010131175 A1

**In der Beschreibung aufgeführte Nicht-Patentliteratur**

- *Medical mycology*, 2017, vol. 55 (3), 334-343
- *Interdisciplinary Toxicology*, 2015, vol. 8 (4), 193-202
- **OWEN L** ; **LAIRD K**. Development of a silver-based dual-function antimicrobial laundry additive and textile coating for the decontamination of healthcare laundry. *J Appl Microbiol.*, 2021, vol. 130 (4), 1012-22